# EUROPÄISCHE PATENTSCHRIFT

(11) **EP 2 379 722 B1**
(45) Veröffentlichungstag und Bekanntmachung des Hinweises auf die Patenterteilung: **07.09.2016**
(21) Anmeldenummer: 09798882.8
(22) Anmeldetag: 15.12.2009
(51) Int. Cl.: C12N 15/70, C12N 15/10

(54) **EXPRESSIONSVEKTOR**
Expression vector
Vecteur d'expression

(30) Priorität: 16.12.2008 EP 08021794
(43) Veröffentlichungstag der Anmeldung: 26.10.2011
(73) Patentinhaber: c-LEcta GmbH, 04103 Leipzig (DE)
(72) Erfinder: GREINER-STÖFFELE, Thomas, 04279 Leipzig (DE); BALLSCHMITTER, Meike, 04275 Leipzig (DE); STRUHALLA, Marc, 04229 Leipzig (DE); CZAJA, Rico, 04157 Leipzig (DE)
(74) Vertreter: Bülle, Jan
(86) Internationale Anmeldenummer: PCT/EP2009/008977
(87) Internationale Veröffentlichungsnummer: WO 2010/075956

(56) Entgegenhaltungen:
- WO-A-00/01846
- WO-A-01/83785
- WO-A-02/083910
- US-A- 6 030 807
- US-A1- 2005 266 026
- CHRISTEL SCHMEISSER ET AL: "Metagenomics, biotechnology with non-culturable microbes" APPLIED MICROBIOLOGY AND BIOTECHNOLOGY, Bd. 75, Nr. 5, 30. März 2007 (2007-03-30), Seiten 955-962, XP019513759 ISSN: 1432-0614 in der Anmeldung erwähnt
- KIM S ET AL: "Rare codon clusters at 5'-end influence heterologous expression of archaeal gene in Escherichia coli" PROTEIN EXPRESSION AND PURIFICATION, Bd. 50, Nr. 1, 1. November 2006 (2006-11-01), Seiten 49-57, XP024908859 in der Anmeldung erwähnt
- STUDIER F W: "USE OF BACTERIOPHAGE T7 LYSOZYME TO IMPROVE AN INDUCIBLE T7 EXPRESSION SYSTEM" JOURNAL OF MOLECULAR BIOLOGY, LONDON, GB, Bd. 219, Nr. 1, 1. Januar 1991 (1991-01-01), Seiten 37-44, XP001069966 ISSN: 0022-2836 in der Anmeldung erwähnt
- LAMMLE ET AL: "Identification of novel enzymes with different hydrolytic activities by metagenome expression cloning", JOURNAL OF BIOTECHNOLOGY, vol. 127, no. 4, 22 December 2006 (2006-12-22), pages 575-592, XP005810971, ISSN: 0168-1656, DOI: 10.1016/J.JBIOTEC.2006.07.036

### SEQUENZPROTOKOLL

<110> Universität Leipzig et al.
<120> Expressionsvektor
<130> IV0005-WO
<160> 4
<170> PatentIn version 3.3
<210> 1
   <211> 2425
   <212> DNA
   <213> Cloning Vector pF2F4
<400> 1
   cgaattcgct agcccaaaaa aacgggtatg gagaaacagt agagagttgc gataaaaagc 60
   gtcaggtagc atccgctaat cttatggata aaaatgctat ggcatagcaa agtgtgacgc 120
   cgtgcaaata atcaatgtgg acttttctgc cgtgattata gacacttttg ttacgcgttt 180
   ttgtcatggc tttggtcccg ctttgttaca gaatgctttt aataagcggg gttaccggtt 240
   tggttagcga gaagagccag taaaagacgc agtgacggca atgtctgatg taatatggac 300
   aattggtttc ttctcagaca attgacggct tgacggagta gcatagggtt tgcagaatcc 360
   ctgcttcgtc catttgacag gcacattatg gcataacccc ttggggcctc taaacgggtc 420
   ttgaggggtt ttttgcttga taagctgtca aacatgagca gatcctctac gccggacgca 480
   tcgtggccct gcattaatga atcggccaac gcgcggggag aggcggtttg cgtattgggc 540
   gctcttccgc ttcctcgctc actgactcgc tgcgctcggt cgttcggctg cggcgagcgg 600
   tatcagctca ctcaaaggcg gtaatacggt tatccacaga atcaggggat aacgcaggaa 660
   agaacatgtg agcaaaaggc cagcaaaagg ccaggaaccg taaaaaggcc gcgttgctgg 720
   cgtttttcca taggctccgc ccccctgacg agcatcacaa aaatcgacgc tcaagtcaga 780
   ggtggcgaaa cccgacagga ctataaagat accaggcgtt tccccctgga agctccctcg 840
   tgcgctctcc tgttccgacc ctgccgctta ccggatacct gtccgccttt ctcccttcgg 900
   gaagcgtggc gctttctcat agctcacgct gtaggtatct cagttcggtg taggtcgttc 960
   gctccaagct gggctgtgtg cacgaacccc ccgttcagcc cgaccgctgc gccttatccg 1020
   gtaactatcg tcttgagtcc aacccggtaa gacacgactt atcgccactg gcagcagcca 1080
   ctggtaacag gattagcaga gcgaggtatg taggcggtgc tacagagttc ttgaagtggt 1140
   ggcctaacta cggctacact agaaggacag tatttggtat ctgcgctctg ctgaagccag 1200
   ttaccttcgg aaaaagagtt ggtagctctt gatccggcaa acaaaccacc gctggtagcg 1260
   gtggtttttt tgtttgcaag cagcagatta cgcgcagaaa aaaaggatct caagaagatc 1320
   ctttgatctt ttctacgggg tctgacgctc agtggaacga aaactcacgt taagggattt 1380
   tggtcatgaa ttaattctta gaaaaactca tcgagcatca aatgaaactg caatttattc 1440
   atatcaggat tatcaatacc atatttttga aaaagccgtt tctgtaatga aggagaaaac 1500
   tcaccgaggc agttccatag gatggcaaga tcctggtatc ggtctgcgat tccgactcgt 1560
   ccaacatcaa tacaacctat taatttcccc tcgtcaaaaa taaggttatc aagtgagaaa 1620
   tcaccatgag tgacgactga atccggtgag aatggcaaaa gtttatgcat ttctttccag 1680
   acttgttcaa caggccagcc attacgctcg tcatcaaaat cactcgcatc aaccaaaccg 1740
   ttattcattc gtgattgcgc ctgagcgaga cgaaatacgc ggtcgctgtt aaaaggacaa 1800
   ttacaaacag gaatcgaatg caaccggcgc aggaacactg ccagcgcatc aacaatattt 1860
   tcacctgaat caggatattc ttctaatacc tggaatgctg ttttcccggg gatcgcagtg 1920
   gtgagtaacc atgcatcatc aggagtacgg ataaaatgct tgatggtcgg aagaggcata 1980
   aattccgtca gccagtttag tctgaccatc tcatctgtaa catcattggc aacgctacct 2040
   ttgccatgtt tcagaaacaa ctctggcgca tcgggcttcc catacaatcg atagattgtc 2100
   gcacctgatt gcccgacatt atcgcgagcc catttatacc catataaatc agcatccatg 2160
   ttggaattta atcgcggcct agagcaagac gtttcccgtt gaatatggct catactcttc 2220
   ctttttcaat attattgaag catttatcag ggttattgtc tcatgagcgg atacatattt 2280
   gaatgtattt agaaaaataa acaaataggg gttccgcgca cattacgtca ttaatacgac 2340
   tcactatagg ggaattgtga gcggataaca attccgatat catgtgcctg caggtcgact 2400
   ctagaggatc cccgggtacc gagct 2425
<210> 2
   <211> 4994
   <212> DNA
   <213> Cloning Vector pLacl_plus
<400> 2
   gaattccgga tgagcattca tcaggcgggc aagaatgtga ataaaggccg gataaaactt 60
   gtgcttattt ttctttacgg tctttaaaaa ggccgtaata tccagctgaa cggtctggtt 120
   ataggtacat tgagcaactg actgaaatgc ctcaaaatgt tctttacgat gccattggga 180
   tatatcaacg gtggtatatc cagtgatttt tttctccatt ttagcttcct tagctcctga 240
   aaatctcgat aactcaaaaa atacgcccgg tagtgatctt atttcattat ggtgaaagtt 300
   ggaacctctt acgtgccgat caacgtctca ttttcgccaa aagttggccc agggcttccc 360
   ggtatcaaca gggacaccag gatttattta ttctgcgaag tgatcttccg tcacaggtat 420
   ttattcggcg caaagtgcgt cgggtgatgc tgccaactta ctgatttagt gtatgatggt 480
   gtttttgagg tgctccagtg gcttctgttt ctatcagctg tccctcctgt tcagctactg 540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttcccctg gcggctccct 960
   cgtgcgctct cctgttcctg cctttcggtt taccggtgtc attccgctgt tatggccgcg 1020
   tttgtctcat tccacgcctg acactcagtt ccgggtaggc agttcgctcc aagctggact 1080
   gtatgcacga accccccgtt cagtccgacc gctgcgcctt atccggtaac tatcgtcttg 1140
   agtccaaccc ggaaagacat gcaaaagcac cactggcagc agccactggt aattgattta 1200
   gaggagttag tcttgaagtc atgcgccggt taaggctaaa ctgaaaggac aagttttggt 1260
   gactgcgctc ctccaagcca gttacctcgg ttcaaagagt tggtagctca gagaaccttc 1320
   gaaaaaccgc cctgcaaggc ggttttttcg ttttcagagc aagagattac gcgcagacca 1380
   aaacgatctc aagaagatca tcttattaat cagataaaat atttctagat ttcagtgcaa 1440
   tttatctctt caaatgtagc acctgaagtc agccccatac gatataagtt gtaattctca 1500
   tgttagtcat gccccgcgcc caccggaagg agctgactgg gttgaaggct ctcaagggca 1560
   tcggtcgaga tcccggtgcc taatgagtga gctaacttac attaattgcg ttgcgctcac 1620
   tgcccgcttt ccagtcggga aacctgtcgt gccagctgca ttaatgaatc ggccaacgcg 1680
   cggggagagg cggtttgcgt attgggcgcc agggtggttt ttcttttcac cagtgagacg 1740
   ggcaacagct gattgccctt caccgcctgg ccctgagaga gttgcagcaa gcggtccacg 1800
   ctggtttgcc ccagcaggcg aaaatcctgt ttgatggtgg ttaacggcgg gatataacat 1860
   gagctgtctt cggtatcgtc gtatcccact accgagatgt ccgcaccaac gcgcagcccg 1920
   gactcggtaa tggcgcgcat tgcgcccagc gccatctgat cgttggcaac cagcatcgca 1980
   gtgggaacga tgccctcatt cagcatttgc atggtttgtt gaaaaccgga catggcactc 2040
   cagtcgcctt cccgttccgc tatcggctga atttgattgc gagtgagata tttatgccag 2100
   ccagccagac gcagacgcgc cgagacagaa cttaatgggc ccgctaacag cgcgatttgc 2160
   tggtgaccca atgcgaccag atgctccacg cccagtcgcg taccgtcttc atgggagaaa 2220
   ataatactgt tgatgggtgt ctggtcagag acatcaagaa ataacgccgg aacattagtg 2280
   caggcagctt ccacagcaat ggcatcctgg tcatccagcg gatagttaat gatcagccca 2340
   ctgacgcgtt gcgcgagaag attgtgcacc gccgctttac aggcttcgac gccgcttcgt 2400
   tctaccatcg acaccaccac gctggcaccc agttgatcgg cgcgagattt aatcgccgcg 2460
   acaatttgcg acggcgcgtg cagggccaga ctggaggtgg caacgccaat cagcaacgac 2520
   tgtttgcccg ccagttgttg tgccacgcgg ttgggaatgt aattcagctc cgccatcgcc 2580
   gcttccactt tttcccgcgt tttcgcagaa acgtggctgg cctggttcac cacgcgggaa 2640
   acggtctgat aagagacacc ggcatactct gcgacatcgt ataacgttac tggtttcaca 2700
   ttcaccaccc tgaattgact ctcttccggg cgctatcatg ccataccgcg aaaggttttg 2760
   cgccattcga tggtgtccgg gatctcgacg ctctccctta tgcgactcct gcattaggaa 2820
   gcagcccagt agtaggttga ggccgttgag caccgccgcc gcaaggaatg gtgtcgtcgc 2880
   cgcacttatg actgtcttct ttatcatgca actcgtagga caggtgcctc aggtagcatc 2940
   cgctaatctt atggataaaa atgctatggc atagcaaagt gtgacgccgt gcaaataatc 3000
   aatgtggact tttctgccgt gattatagac acttttgtta cgcgtttttg tcatggcttt 3060
   ggtcccgctt tgttacagaa tgcttttaat aagcggggtt accggtttgg ttagcgagaa 3120
   gagccagtaa aagacgcagt gacggcaatg tctgatgcaa tatggacaat tggtttcttc 3180
   tctgaatggc gggagtatga aaagtatggc tgaagcgcaa aatgatcccc tgctgccggg 3240
   atactcgttt aatgcccatc tggtggcggg tttaacgccg attgaggcca acggttatct 3300
   cgattttttt atcgaccgac cgctgggaat gaaaggttat attctcaatc tcaccattcg 3360
   cggtcagggg gtggtgaaaa atcagggacg agaatttgtt tgccgaccgg gtgatatttt 3420
   gctgttcccg ccaggagaga ttcatcacta cggtcgtcat ccggaggctc gcgaatggta 3480
   tcaccagtgg gtttactttc gtccgcgcgc ctactggcat gaatggctta actggccgtc 3540
   aatatttgcc aatacggggt tctttcgccc ggatgaagcg caccagccgc atttcagcga 3600
   cctgtttggg caaatcatta acgccgggca aggggaaggg cgctattcgg agctgctggc 3660
   gataaatctg cttgagcaat tgttactgcg gcgcatggaa gcgattaacg agtcgctcca 3720
   tccaccgatg gataatcggg tacgcgaggc ttgtcagtac atcagcgatc acctggcaga 3780
   cagcaatttt gatatcgcca gcgtcgcaca gcatgtttgc ttgtcgccgt cgcgtctgtc 3840
   acatcttttc cgccagcagt tagggattag cgtcttaagc tggcgcgagg accaacgtat 3900
   cagccaggcg aagctgcttt tgagcaccac ccggatgcct atcgccaccg tcggtcgcaa 3960
   tgttggtttt gacgatcaac tctatttctc gcgggtattt aaaaaatgca ccggggccag 4020
   cccgagcgag ttccgtgccg gttgtgaaga aaaagtgaat gatgtagccg tcaagttgtc 4080
   ataattggta acgaatcaga caattgacgg gcagcgccca acagtccccc ggccacgggg 4140
   cctgccacca tacccacgcc gaaacaagcg ccctgcacca ttatgttccg gatctgcatc 4200
   gcaggatgct gctggctacc ctgtggaaca cctacatctg tattaacgaa gcgctaaccg 4260
   tttttatcag gctctgggag gcagaataaa tgatcatatc gtcaattatt acctccacgg 4320
   ggagagcctg agcaaactgg cctcaggcat ttgagaagca cacggtcaca ctgcttccgg 4380
   tagtcaataa accggtaaac cagcaataga cataagcggc tatttaacga ccctgccctg 4440
   aaccgacgac cgggtcgaat ttgctttcga atttctgcca ttcatccgct tattatcact 4500
   tattcaggcg tagcaccagg cgtttaaggg caccaataac tgccttaaaa aaattacgcc 4560
   ccgccctgcc actcatcgca gtactgttgt aattcattaa gcattctgcc gacatggaag 4620
   ccatcacaga cggcatgatg aacctgaatc gccagcggca tcagcacctt gtcgccttgc 4680
   gtataatatt tgcccatggt gaaaacgggg gcgaagaagt tgtccatatt ggccacgttt 4740
   aaatcaaaac tggtgaaact cacccaggga ttggctgaga cgaaaaacat attctcaata 4800
   aaccctttag ggaaataggc caggttttca ccgtaacacg ccacatcttg cgaatatatg 4860
   tgtagaaact gccggaaatc gtcgtggtat tcactccaga gcgatgaaaa cgtttcagtt 4920
   tgctcatgga aaacggtgta acaagggtga acactatccc atatcaccag ctcaccgtct 4980
   ttcattgcca tacg 4994
<210> 3
   <211> 13
   <212> RNA
   <213> mammal
<220>
   <221> misc_feature
   <222> (7)..(7)
   <223> n is a or g
<400> 3
   gccgccncca ugg 13
<210> 4
   <211> 12
   <212> RNA
   <213> yeast
<220>
   <221> misc_feature
   <222> (1)..(1)
   <223> n is a or u
<220>
   <221> Intron
   <222> (2)..(2)
   <223> test
<220>
   <221> misc_feature
   <222> (3)..(3)
   <223> n is a or c
<220>
   <221> misc_feature
   <222> (6)..(6)
   <223> n is a or c
<220>
   <221> misc_feature
   <222> (12)..(12)
   <223> n is u or c
<400> 4
   nanaanaugu cn 12

## Beschreibung

Die Erfindung betrifft einen Expressionsvektor, welcher sich zur effizienten Durchmusterung (*Screening*) von (Meta)genom-Bibliotheken eignet, vorzugsweise in *Escherichia. coli.*

Nur etwa 1-5% aller bekannten Mikroorganismen sind heute mit gängigen Methoden im Labor kultivierbar. In letzter Zeit wurden Verfahren entwickelt, welche die genetischen Ressourcen der nicht-kultivierbaren Mikroorganismen nutzbar machen sollen. Dieses Gebiet wird auch als "Metagenomik" bezeichnet, wobei der Begriff "Metagenom" die genetische Information sämtlicher Organismen eines bestimmten Habitats bezeichnet, unabhängig davon, ob diese kultivierbar sind oder nicht.

Durch direktes Klonieren der aus Umweltproben gewonnen DNA in geeignete Vektorsysteme (Plasmide, Cosmide, BACs, YACs) steht diese Ressource im Labor leicht handhabbar zur Verfügung. Diese Genbanken (Metagenom-Bibliotheken) können z.B. für die Suche nach neuartigen Enzymen genutzt werden. Um völlig neuartige Enzymaktivitäten aufzufinden, ist eine aktivitätsbasierte Durchmusterung erstellter Metagenom-Bibliotheken erforderlich. Voraussetzung hierfür ist ein geeignetes Nachweissystem (Agarplattentests, Mikrotiterplattensysteme), das zugleich eine Durchmusterung einer möglichst großen Zahl an Klonen erlaubt *(high throughput screening*). Des Weiteren muss die Expression der Gene in einem heterologen Wirt gewährleistet sein. Neben *E*. *coli* werden in Metagenomstudien auch andere Organismen wie *Streptomyces lividans* oder *Pseudomonas putida* als Wirt herangezogen.

Probleme der Metagenomtechnik liegen insbesondere in der Expression der gefundenen Gene. Hier sind es mangelnde Transkription, da z.B. Promotoren nicht erkannt werden, Toxizität der Produkte für den Wirt, fehlende Cofaktoren oder Faltungshelfer und somit unkorrekte Faltung der Proteine im heterologen Wirt, sowie fehlende Sekretionssysteme (W.R. Streit et al., Curr Opin Microbiol. 2004, 7(5), 492-8).

Herkömmliche (Meta)genom-Bibliotheken für die Durchmusterung in *E*. *coli* werden meist in artifiziellen Chromosomen (BAC), Cosmid- bzw. Fosmid-Systemen oder Plasmiden konstruiert. Bisher werden für die Erstellung (meta)genomischer Plasmid-Bibliotheken überwiegend herkömmliche Klonierungsvektoren verwendet, welche meist einen einzelnen, vergleichsweise schwachen Promotor (z.B. lac-Promotor) aufweisen oder vollständig auf die Nutzung interner Promotoren der klonierten DNA ausgelegt sind. Dieser schwache Promotor war ursprünglich nicht für die Expression der klonierten DNA gedacht, sondern liegt als Promotor vor dem häufig als Marker verwendeten lacZ-Gen. In diesem Zusammenhang kann beispielsweise verwiesen werden auf R. Ranjan et al., Biochem Biophys Res Commun., 2005, 335(1), 57-65; und A. Knietsch et al., Appl Environ Microbiol., 2003, 69(3), 1408-1416.

Bei einem sequenzbasierten Screening der (Meta)genom-Bibliothek hat die relative Schwäche des Promotors keine negativen Folgen. Werden die gleichen Plasmidbibliotheken jedoch zum Screening der Aktivität der durch die Bibliothek codierten Zielproteine eingesetzt, so beruht die Expression der Zielproteine oft auf dem plasmidständigen schwachen Promotor. Bei den häufig verwendeten Cosmid/Fosmid Systemen beruht die funktionelle Expression der Zielgene ausschließlich auf der Erkennung und dem Ablesen der auf der Insert-DNA liegenden *E*. *coli*-fremden Promotoren. In diesem Zusammenhang kann beispielsweise verwiesen werden auf K.S. Hong et al., J Microbiol Biotechnol., 2007, 17(10), 1655-60.

Durch die Schwäche des Promotors bzw. das Nichterkennen *E*.*coli*-fremder Promotoren wird ein Teil der Zielproteine kaum oder gar nicht exprimiert, wodurch ein Aktivitätsscreening der Zielproteine erheblich erschwert wird. Diese Einschränkungen machen in den meisten Fällen ein iteratives Aktivitätsscreening von Sub-Bibliotheken (Cluster-Screening, vgl. WO 2005/ 040376) unmöglich. Stattdessen wird ein umständliches und zeitaufwändiges Aktivitätsscreening mit Einzelklonen z.B. auf Agarplatten notwendig.

Ein weiteres Problem beim Aktivitätsscreening besteht darin, dass beim Erstellen von (Meta)genom-Bibliotheken die Orientierung des offenen Leserrasters (*open reading frames*, ORF) auf der klonierten DNA nicht beeinflussbar ist. Möglich ist auch, dass zwei aufeinander folgende offene Leseraster unterschiedliche Leserichtungen haben. Beim Aktivitätsscreening mit herkömmlichen Expressionsvektoren geht daher häufig ein großer Teil der in der (Meta)-genom-Bibliothek enthaltenen Sequenzinformation verloren, da der verwendete Promotor lediglich eine der beiden möglichen Leserichtungen erfasst.

WO 01/83785 offenbart ein reguliertes System zum Liefern von Antigenen. Im System steht jedoch die in die Insertionssequenz zu klonierende DNA nicht unter der Kontrolle von beiden Promotoren. Vielmehr kontrolliert einer der beiden Promotoren den *ori* bzw. ein Gen zur Regulation des *ori.* Zum Durchmustern von Metagenom-Bibliotheken eignet sich ein solches System kaum, da nur 50% der enthaltenen Sequenzinformation erfasst wird.

US 6,030,807 offenbart ein Operon, welche für Enzyme kodiert, die mit der Verwendung von L-Arabinose verknüpft sind. Das Operon weist jedoch keine Insertionssequenz auf, welche zwischen zwei aufeinander zulaufenden Promotoren liegt. Das System umfasst auch keinen Vektor mit zwei unterschiedlichen aufeinander zulaufenden Promotoren, zwischen denen jeweils stromabwärts eine Insertionssequenz angeordnet ist.

WO 02/083910 offenbart ein Verfahren zur Herstellung eines Vektors, der wenigstens ein spleißbares Intron einschließt. Der Vektor ist jedoch nicht maximal 3000 bp groß.

Schmeisser et al., Appl. Microbiol. Biotechnol 2007, 75(5), 955-62 ist ein Review zum Thema Metagenomics, Biotechnologie mit nicht-kultivierbaren Mikroben. Die Druckschrift enthält keinerlei Hinweise auf die Expression in Plasmiden mit zwei aufeinander zulaufenden, getrennt voneinander induzierbare Promotoren, zwischen denen jeweils stromabwärts eine Insertionssequenz angeordnet ist, so dass die Expression einer in die Insertionssequenz klonierten DNA-Sequenz unter die Kontrolle der beiden Promotoren gestellt wird.

WO 00/01846 offenbart ein Verfahren zum Identifizieren von DNA, welche für einen bestimmten Phänotyp verantwortlich ist. Bei dem Verfahren wird jedoch kein Vektor mit getrennt voneinander induzierbaren, aufeinanderzulaufenden Promotoren verwendet. Es ist sogar Voraussetzung des Verfahrens, dass beide Promotoren gleich sind. Der Vektor umfasst nicht höchstens 3000 bp.

S. Kim et al., Prot. Expr Purif. 2006, 50(1), 49-57 offenbart seltene Kodon-Cluster am 5'-Terminus, welche die heterologe Expression von archäischen Genen in *E*. *coli* beeinflussen. Die Druckschrift enthält jedoch keinerlei Hinweise auf einen Expressionsvektor, der zwei getrennt voneinander induzierbare, aufeinander zulaufende Promotoren umfasst, zwischen denen jeweils stromabwärts eine Insertionssequenz angeordnet ist, so dass die Expression einer in die Insertionssequenz klonierten DNA-Sequenz unter die Kontrolle der beiden Promotoren gestellt wird.

F.W. Studier, J. Mol. Biol. 1991, 219(1), 37-44 offenbart die Verwendung von Bakteriophagen T7 Lysozym zur Verbesserung eines induzierbaren T7 Expressionssystems. Das System weist jedoch keinen Expressionsvektor auf, der zwei getrennt voneinander induzierbare, aufeinander zulaufende Promotoren umfasst, zwischen denen jeweils stromabwärts eine Insertionssequenz angeordnet ist, so dass die Expression einer in die Insertionssequenz klonierten DNA-Sequenz unter die Kontrolle der beiden Promotoren gestellt wird.

Es ist eine Aufgabe der Erfindung, ein Expressionssystem bereit zu stellen, welches sich zur Durchmusterung, insbesondere zum Aktivitätsscreening, von (Meta)genom-Bibliotheken eignet und Vorteile gegenüber den Systemen des Standes der Technik aufweist. Das Expressionssystem sollte sich möglichst durch eine hohe Klonierungseffizienz verbunden mit effizienter, regulierbarer Expression auszeichnen und einen möglichst großen Anteil der in der (Meta)genom-Bibliothek enthaltenen Sequenzinformation erfassen.

Diese Aufgabe wird durch den Gegenstand der Patentansprüche gelöst.

Ein erster Aspekt der Erfindung betrifft einen Expressionsvektor umfassend zwei getrennt voneinander induzierbare, aufeinander zulaufende Promotoren P₁ und P₂, wobei vorzugsweise zwischen P₁ und P₂ jeweils stromabwärts eine Insertionssequenz angeordnet ist, so dass die Expression einer in die Insertionssequenz klonierten DNA-Sequenz unter die Kontrolle von P₁ und P₂ gestellt wird; wobei die Insertionssequenz ein Polylinker und/oder eine Sequenz ist, welche eine Integration von DNA-Sequenzen mittels Rekombination ermöglicht; wobei der Expressionsvektor ohne Insertionssequenz insgesamt höchstens 3000 bp umfasst; und wobei der Expressionsvektor nicht für einen Regulator R₁ von P₁ und nicht für einen Regulator R₂ von P₂ kodiert.

In diesem Zusammenhang bedeutet "unter der Kontrolle von P₁ und P₂", dass die Expression der klonierten, doppelsträngigen DNA-Sequenz durch P₁ und P₂ kontrolliert werden kann. Dabei wird der eine Strang der klonierten, doppelsträngigen DNA-Sequenz durch P₁ und der dazu komplementäre Strang der klonierten, doppelsträngigen DNA-Sequenz durch P₂ kontrolliert. Die Kontrolle erfolgt bevorzugt im Sinne eines Operons.

Es wurde überraschend gefunden, dass sich der erfindungsgemäße Expressionsvektor besonders gut zum Aktivitätsscreening von (Meta)genom-Bibliotheken eignet, da beide Leserichtungen erfasst werden. Der Verlust der Hälfte der in der (Meta)genom-Bibliothek enthaltenen Sequenzinformation bzw. die Notwendigkeit die doppelte Anzahl von Klonen durchmustern zu müssen, wie es bei Verwendung herkömmlicher Expressionsvektoren hingenommen werden muss, kann mit Hilfe des erfindungsgemäßen Expressionsvektors vermieden werden.

Vorzugsweise handelt es sich um einen Expressionsvektor für *E*. *coli,* bei dem zwei starke Promotoren die *multiple cloning site* flankieren. Die Promotoren sind konvergent, d.h. ihre Leserichtung ist aufeinander zulaufend (*face-to-face*). Bei den unabhängig voneinander

induzierbaren Promotoren handelt es sich vorzugsweise um einen T7-Promotor und einen ara-Promotor.

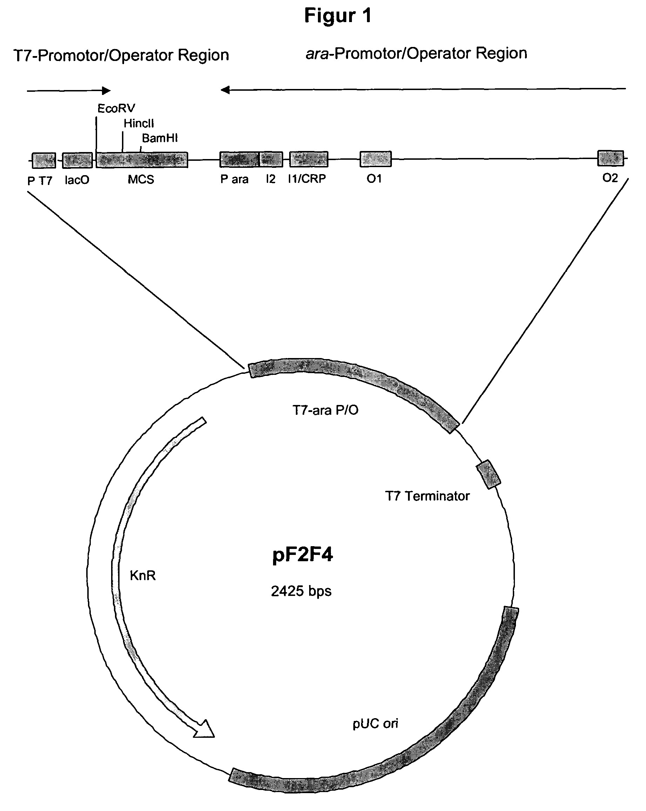

Figur 1 zeigt pF2F4, eine bevorzugte Ausführungsform eines erfindungsgemäßen Expressionsvektors mit <SEQ.ID.NO: 1>. Es handelt sich um einen Expressionsvektor für *E*. *coli,* bei dem zwei starke Promotoren die *multiple cloning site* flankieren. Die Promotoren sind konvergent, d.h. ihre Leserichtung ist aufeinander zulaufend (*face-to-face*). Bei den unabhängig voneinander induzierbaren Promotoren handelt es sich um einen T7-Promotor und einen Arabinose-Promotor.

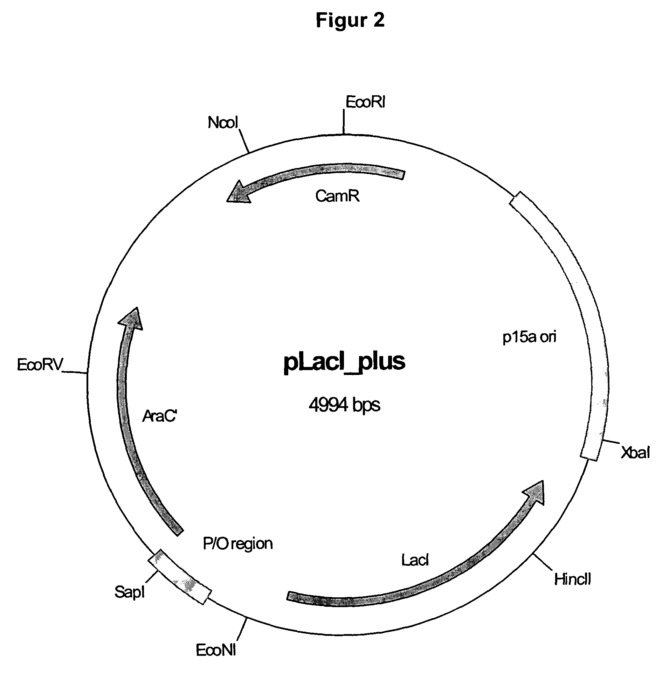

Figur 2 zeigt das Regulatorplasmid pLacl+ mit <SEQ.ID.NO: 2>, mit welchem erfindungsgemäß bevorzugt der Wirtsorganismus zusammen mit dem Expressionsvektor transformiert wird.

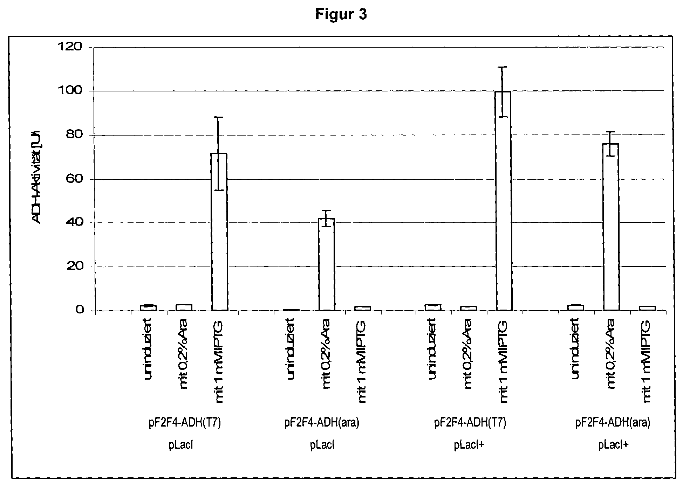

Figur 3 zeigt im Zusammenhang mit Beispiel 1 pF2F4 mit verschieden orientierter Alkohol-dehydrogenase als Reportergen in *E*. *coli* BL21(DE3) Zellen, in denen gleichzeitig pLacI bzw. pLacl+ propagiert wird. Alle Messungen der bei T7-Induktion mit 1% Glucose im Medium, der Ara-Induktion ohne weiteren Glucose Zusatz.

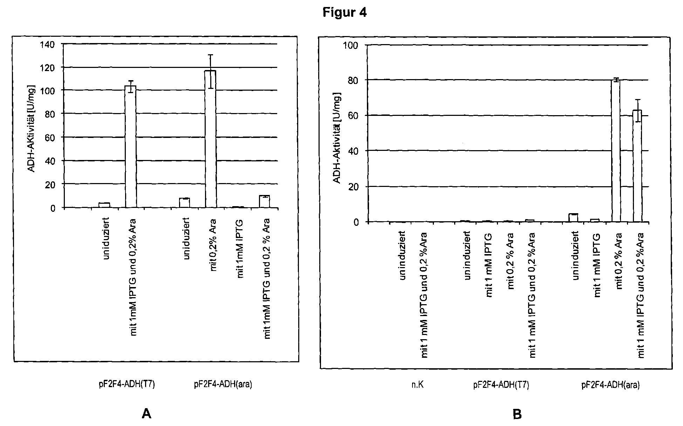

Figur 4A zeigt im Zusammenhang mit Beispiel 1 pF2F4 mit verschieden orientierter Alkohol-Dehydrogenase als Reportergen in *E*. *coli* BL21(DE3). Figur 4B zeigt pF2F4 mit verschieden orientierter Alkohol-Dehydrogenase als Reportergen in *E*. *coli* DH10B. In allen Ansätzen wurde das Plasmid pLacI+ coexprimiert.

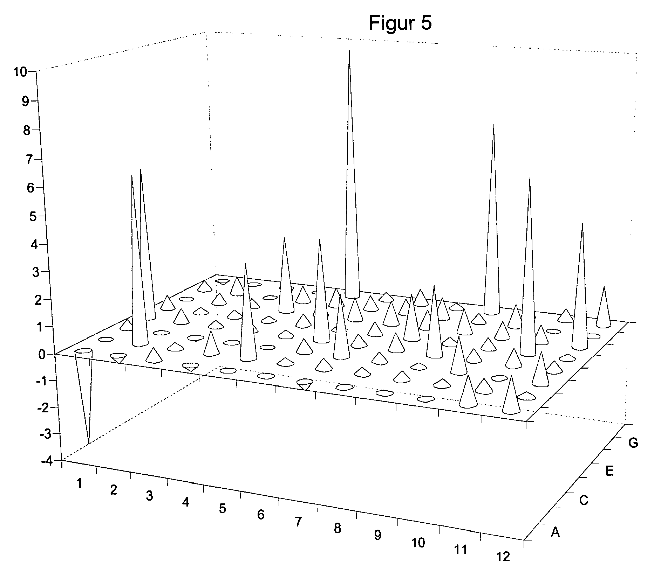

Figur 5 zeigt im Zusammenhang mit Beispiel 2 die Hitverteilung nach 3 h Inkubationszeit im IPTG-induzierten Zellextrakt der Pansenbibliothek in pF2F4. A1 ist als Kontrolle unbeimpft.

Ein Expressionsvektor im Sinne der vorliegenden Erfindung stellt vorzugsweise eine DNA-Sequenz dar, welche mindestens eine DNA-Sequenz zur Replikation in Wirten (*origin of replication*); mindestens eine DNA-Sequenz kodierend für eine Sequenz, welche geeignet ist, Wirte, die den Expressionsvektor enthalten, von Wirten zu unterscheiden, die den Expressionsvektor nicht enthalten (im Rahmen der vorliegenden Erfindung als "Selektionsmarkersequenz" bezeichnet); mindestens einer DNA-Sequenz zur Insertion von Fremd-DNA (im Rahmen der vorliegenden Erfindung als "Insertionssequenz" bezeichnet), und mindestens eine DNA-Sequenz, welche von einer RNA-Polymerase als Transkriptionsstartpunkt erkannt wird, umfasst.

Der erfindungsgemäße Expressionsvektor eignet sich zur Expression von Peptiden bzw. Proteinen in prokaryotischen oder eukaryotischen Systemen (Wirten).

Bevorzugte prokaryotische Systeme umfassen z.B. Bakterien. Bevorzugte Bakterien umfassen *E*. *coli, Bacillus sp., Salmonelly typhimurium, Staphylococcus sp., Pseudomonas sp., Streptomyces* sp. und *Caulobacter sp.* und *Borrelia sp..* Bevorzugte eukaryotische Systeme umfassen z.B. Hefen oder SF9-Zellen, Chinese Hamster Ovary Zellen, und andere Zellen höherer Organismen. Bevorzugte Hefen umfassen *Saccharomyces cerevisiae, Schizosaccharomyces pombe* und *Pichia pastoris.*

Für die Auswahl des Wirtes können verschiedene Aspekte eine Rolle spielen. Ein wichtiger Aspekt ist die Möglichkeit der posttranslationalen Modifikation des exprimierten Peptides/ Proteins in der Wirtszelle. Ein anderer Aspekt ist die Eignung der Wirtszelle zur Sekretion der exprimierten Peptide/Proteine. In Abhängigkeit von der biologischen Quelle der (Meta)-genom-Bibliothek kann ein Fachmann entscheiden, welcher Wirt zur Expression am geeignetsten erscheint. Bevorzugt ist die biologische Quelle der (Meta)genom-Bibliothek rein prokaryotischen Ursprungs, rein eukaryotischen Ursprungs oder gemischt prokaryotischen und eukaryotischen Ursprungs. Die Quelle kann beispielsweise einer maritimen oder terrestrischen Umgebung entstammen. Beispiele für geeignete Quellen können Organismen sein, welche in natürlicher oder in artifizieller, insbesondere human-beeinflusster Umgebung leben. In diesem Zusammenhang kommen auch vergleichsweise extreme Umgebungen in Betracht, wie z.B. Vulkane, heiße Quellen, Wüsten, Eislandschaften, Gletscher, Gebiete mit ungewöhnlich hohen oder niedrigen pH-Werten, Gebiete mit hoher Strahlenbelastung oder sonstige umweltbelastete Biotope. In einer bevorzugten Ausführungsform entstammen die Quellen Kläranlagen, Biofiltern oder sonstigen technischen Anlagen.

Vorzugsweise ist der erfindungsgemäße Expressionsvektor ein Plasmid, z.B. ein bakterielles Plasmid oder ein Hefeplasmid.

In einer bevorzugten Ausführungsform handelt es sich bei dem erfindungsgemäßen Expressionsvektor um ein *low-copy* Plasmid (durchschnittlich < 100 Plasmide pro Zelle). In einer anderen bevorzugten Ausführungsform handelt es sich bei dem erfindungsgemäßen Expressionsvektor um ein *high-copy* Plasmid (durchschnittlich ≥ 100 Plasmide pro Zelle).

Relevant für die Anzahl der Kopien des (nicht in das Chromosom integrierten) Expressionsvektors pro Zelle ist das verwendete *origin of replication* (*ori*). Dem Fachmann ist eine Vielzahl von *ori* bekannt und er ist in der Lage, ein für eine jeweils bevorzugte Ausführungsform geeignetes *ori* auszuwählen. Beispielsweise können folgende *ori* oder auf den folgenden *ori* basierenden *ori* verwendet werden: *E.coli oriC, ColE1-ori* oder die *ori* aus verschiedenen, dem Fachmann bekannten Plasmiden wie pUC, pBR322, pGEM, pTZ, pBlueskript, pMB1, pSC101, p15a, pR6K, das M13-*ori*, oder, zur Expression in Hefezellen, der 2 µm *ori* oder, zur Expression in andern eukaryotischen Wirten, *ori* wie der SV40-*ori*.

Erfindungsgemäß kann der Expressionsvektor, insbesondere das Expressionsplasmid, auch mehrere *ori*, beispielsweise 2 *ori's* enthalten. Hierbei kann es sich beispielsweise um eine Kombination aus einem *low copy ori* und einem temperaturabhängigen *ori* handeln oder beispielsweise um *ori's,* welche das propagieren in verschiedenen Wirtsorganismen erlauben (*ori* für *E*. *coli* und *ori* für *Bacillus* sp.).

Neben Plasmiden kommen als erfindungsgemäßer Expressionsvektor auch andere Vektoren in Betracht, beispielsweise Phagen, Cosmide, Phagen, Phasmids, Fosmide, Bacterial Artificial Chromsomes, Yeast Artificial Chromosomes, Viren und Retroviren (beispielsweise Vaccinia, Adenovirus, Adeno-associated Virus, Lentivirus, Herpes-Simplex-Virus, Epstein-Barr-Virus, Fowl Pox Virus, Pseudorabies, Baculovirus) und daraus abgeleitete Vektoren.

Der Expressionsvektor oder Teile davon können auch in das Genom integriert werden.

Jeder andere Vektor kann zur Herstellung des erfindungsgemäßen Expressionsvektors eingesetzt werden, solange er replizierbar und überlebensfähig im gewählten System (Wirt) ist.

Je nach (Meta)genom-Bibliothek und zur Expression geeignet erscheinendem Wirt erfolgt bevorzugt die Auswahl der Promotoren P₁ und P₂ auf einem geeigneten Vektor.

Erfindungsgemäß umfasst der Begriff "Promotor" jede Transkriptionskontrollsequenz, welche es ermöglicht, ein Peptid oder Protein in einem geeigneten System zu exprimieren, d.h. die codierte DNA-Sequenz in RNA zu transkribieren und anschließend in die entsprechende Peptid- bzw. Proteinsequenz zu translatieren. Demzufolge umfasst der Begriff nicht nur die Promotor-Sequenz als solche (die Bindungsstelle der RNA-Polymerase), sondern ggf. zusätzlich auch die Verstärker-Sequenz (Enhancer-Sequenz), die Operator-Sequenz, und dergleichen.

Als Promotoren P₁ und P₂ kommen erfindungsgemäß grundsätzlich alle Nukleotidsequenzen in der DNA des Expressionsvektors in Betracht, an welche RNA-Polymerasen binden, um eine Transkription zu beginnen. Vorzugsweise handelt es sich dabei um RNA-Polymerase nativer, natürlich vorkommenden Organismen, z.B. *E. coli.* Der Begriff umfasst in Bezug auf einen gegebenen Wirt auch solche Promotoren, an welche RNA-Polymerase anderer Organismen binden. So kann beispielsweise die RNA-Polymerase des T7-Bakteriophagen in *E*. *coli* mitexprimiert werden, um den T7-Promotor in *E*. *coli* nutzen zu können, z.B. in *E coli* BL21(DE3).

Im Rahmen der vorliegenden Erfindung bezeichnet "Pᵢ" wahlweise P₁ oder P₂.

In einer bevorzugten Ausführungsform handelt es sich bei P₁ und P₂ um prokaryotische Promotoren. In einer anderen bevorzugten Ausführungsform handelt es sich bei P₁ und P₂ um eukaryotische Promotoren.

In einer bevorzugten Ausführungsform können P₁ und P₂ jeweils beide vom selben Organismus adressiert werden, d.h. können ihre Funktionalität im selben Organismus entfalten und sind mit demselben Organismus kompatibel. Befindet sich der erfindungsgemäße Expressionsvektor beispielsweise in einem bestimmten Mikroorganismus, so können bevorzugt beide Promotoren P₁ und P₂ von den in diesem Mikroorganismus enthaltenen RNA-Polymerasen erkannt werden; es sind dazu bevorzugt keine weiteren Organismen erforderlich.

Prokaryotische Promotoren umfassen üblicherweise ein sog. "-35 Element" und die sog. "TATA-Box" bzw. *"Pribnow-Box".* Die Consensus-Sequenz für das -35 Element umfasst die folgenden sechs Nukleotide: TTGACA. Die Consensus-Sequenz für die *Pribnow*-Box umfasst die sechs Nukleotide TATAAT. In einer bevorzugten Ausführungsform unterscheiden sich die beiden Promotoren P₁ und P₂ in mindestens 1 Nukleotid innerhalb der Gesamtheit dieser beiden Sequenzabschnitte, bevorzugter in mindestens 2 Nukleotiden, noch bevorzugter mindestens 3 Nukleotiden, am bevorzugtesten mindestens 4 Nukleotiden und insbesondere mindestens 5 Nukleotiden. In einer anderen bevorzugten Ausführungsform unterscheiden sich die beiden Promotoren P₁ und P₂ in höchstens 5 Nukleotiden innerhalb der Gesamtheit dieser beiden Sequenzabschnitte, bevorzugter höchstens 4 Nukleotiden, noch bevorzugter höchstens 3 Nukleotiden, am bevorzugtesten höchstens 2 Nukleotiden und insbesondere höchstens 1 Nukleotid.

In einer bevorzugten Ausführungsform unterscheidet sich der Promotor P₁ in wenigstens 1 Nukleotid, bevorzugter in mindestens 2 Nukleotiden, noch bevorzugter mindestens 3 Nukleotiden, am bevorzugtesten mindestens 4 Nukleotiden und insbesondere mindestens 5 Nukleotiden von der Gesamtheit der beiden oben genannten Consensussequenzen. In einer anderen bevorzugten Ausführungsform unterscheidet sich der Promotor P₁ in höchstens 5 Nukleotiden, bevorzugter höchstens 4 Nukleotiden, noch bevorzugter höchstens 3 Nukleotiden, am bevorzugtesten höchstens 2 Nukleotiden und insbesondere höchstens 1 Nukleotiden von der Gesamtheit der beiden oben genannten Consensussequenzen.

In einer bevorzugten Ausführungsform unterscheidet sich außerdem der Promotor P₂ in wenigstens 1 Nukleotid, bevorzugter in mindestens 2 Nukleotiden, noch bevorzugter mindestens 3 Nukleotiden, am bevorzugtesten mindestens 4 Nukleotiden und insbesondere mindestens 5 Nukleotiden von der Gesamtheit der beiden oben genannten Consensussequenzen. In einer anderen bevorzugten Ausführungsform unterscheidet sich außerdem der Promotor P₂ in höchstens 5 Nukleotiden, bevorzugter höchstens 4 Nukleotiden, noch bevorzugter höchstens 3 Nukleotiden, am bevorzugtesten höchstens 2 Nukleotiden und insbesondere höchstens 1 Nukleotiden von der Gesamtheit der beiden oben genannten Consensussequenzen.

Einen Einfluss auf die Stärke des Promotors hat auch der Abstand zwischen der TATA-Box und der "-35-Box". Vorzugsweise beträgt der Abstand zwischen der TATA-Box und der "-35-Box" des Promotors P₁ 5 bis 50 bp, bevorzugter 10 bis 30 bp, noch bevorzugter 12 bis 25 bp, noch bevorzugter 15 bis 20 bp, am bevorzugtesten 17 bp. Vorzugsweise beträgt der Abstand zwischen der TATA-Box und der "-35-Box" des Promotors P₂ 5 bis 50 bp, bevorzugter 10 bis 30 bp, noch bevorzugter 12 bis 25 bp, noch bevorzugter 15 bis 20 bp, am bevorzugtesten 17 bp.

Vorzugsweise sind P₁ und P₂ extern reguliert, d.h. es handelt sich um funktionelle Promotoren, deren Aktivität durch wenigstens ein weiteres Element (Molekül, Komponente, CoFaktor, Transkriptionsfaktor, etc.) verändert werden kann (erhöht bzw. verringert).

Geeignete Promotoren und deren Teilsequenzen sind dem Fachmann bekannt. Beispiele für geeignete Promotoren umfassen virale, pflanzliche, bakterielle, fungale, humane und tierische Promotoren, z.B. *cos-, tac-, trp-, tet-, trp-tet-, lpp-, lac-, lpp-lac-, laclq-,* T7-, T5-, T3-, *gal-, trc-, ara-,* SP6-, I-PR- oder im I-PL-Promotoren oder deren Teilsequenzen, welche vorzugsweise in gram-negativen Bakterien Anwendung finden. Weitere vorteilhafte Promotoren sind beispielsweise in den gram-positiven Promotoren wie *amy, npr, apr* und SP02, in den Hefepromotoren wie ADC1, MFa, AC, P-60, CYC1, GAPDH oder in Mammaliapromotoren wie CaM-Kinase II, CMV, Nestin, L7, BDNF, NF, SV40, RSV, HSV-TK, Metallothionein-Gen, MBP, NSE, beta-Globin, GFAP, GAP43, Tyrosin Hydroxylase, Kainat-Rezeptor-Untereinheit 1, Glutamat-Rezeptor- Untereinheit B enthalten. Prinzipiell können alle natürlichen Promotoren wie die oben genannten verwendet werden. Darüberhinaus können auch synthetische Promotoren vorteilhaft verwendet werden.

Bevorzugt ist P₁ ≠ P₂.

In einer bevorzugten Ausführungsform ist einer der beiden Promotoren P₁ und P₂ intrinsisch im Hinblick auf den verwendeten Wirt, d.h. zumindest eine intrinsische RNA-Polymerase des Wirts ist in der Lage, an den Promotor zu binden und eine Transkription zu katalysieren, und der andere Promotor ist extrinsisch im Hinblick auf den verwendeten Wirt, d.h. keine intrinsische RNA-Polymerase des Wirts ist in der Lage, an den Promotor zu binden und eine Transkription zu katalysieren. In diesem Zusammenhang bedeutet extrinsisch, dass der Wildtyp des Wirts für diese RNA-Polymerase nicht codiert. In diesem Zusammenhang bedeutet "Transkription katalysieren", dass die intrinsischen RNA-Polymerasen des Wirtes in einem entsprechenden *in*-*vitro*-Transkriptionsassay höchstens 10%, bevorzugt höchstens 1%, bevorzugter höchstens 0,1% der Transkriptionsrate erzielt wie die für diesen Promotor vorliegende extrinsische RNA-Polymerase. In dieser Ausführungsform wird die entsprechend erforderliche extrinsische RNA-Polymerase coexprimiert.

In einer bevorzugten Ausführungsform ist die Genexpression durch P₁ durch einen einzelnen spezifischen Faktor reguliert, nämlich durch den Regulator R₁. In einer anderen bevorzugten Ausführungsform ist die Genexpression durch P₁ durch mindestens zwei spezifische Faktoren reguliert, nämlich durch die Regulatoren R₁^{a} und R₁^{b}, wobei R₁^{a} beispielsweise ein Repressor und R₁^{b} beispielsweise ein Aktivator sein kann. Analoges gilt für P₂ und R₂ bzw. R₂^{a} und R₂^{b}.

In einer bevorzugten Ausführungsform (a) erfordert es der Promotor P₁ und/oder der Promotor P₂, dass zur Bindung der RNA-Polymerase an die entsprechende Erkennungssequenz des Promotors ein Regulator R₁ bzw. R₂ an den Promotor gebunden ist, d.h. die Transkription erfolgt, solange eine Bindung von R₁ an P₁ bzw. von R₂ an P₂ vorliegt.

In einer anderen bevorzugten Ausführungsform (b) erfordert es der Promotor P₁ und/oder der Promotor P₂, dass zur Bindung der RNA-Polymerase an die entsprechende Erkennungssequenz des Promotors ein Regulator R₁ bzw. R₂ nicht an den Promotor gebunden ist, d.h. die Transkription erfolgt, solange keine Bindung von R₁ an P₁ bzw. von R₂ an P₂ vorliegt. Ein Beispiel für eine solche Wechselwirkung von Promotor und Regulator ist die Wechselwirkung eines um mindestens eine lacO-Operator-Sequenz erweiterten T7-Promotors in Kombination mit dem Repressor *LacI.*

In einer weiteren Ausführungsform (c) erfordert es der Promotor P₁ und/oder der Promotor P₂, dass zur Bindung der RNA-Polymerase an die entsprechende Erkennungssequenz des Promotors ein Regulator R₁ bzw. R₂ an den Promotor gebunden ist, der Regulator R₁ bzw. R₂ jedoch unterschiedliche Konformationen einnehmen kann, ohne dass dadurch die Bindung an den Promotor dauerhaft aufgehoben wird, und die Transkription nur dann erfolgt, solange R₁ bzw. R₂ in *einer* der möglichen Konformationen vorliegt. Ein Beispiel für eine solche Wechselwirkung von Promotor und Regulator ist die Wechselwirkung des ara-Promotors mit seinem Aktivator/Repressor *AraC.*

Vorzugsweise gehören die Promotoren P₁ und P₂ unterschiedlichen dieser Ausführungsformen (a), (b) und (c) an, besonders bevorzugt (a) und (c).

Bevorzugt wird das System P₁ / R₁ und/oder das System P₂ / R₂ durch ein weiteres Element I₁ / I₂ (Induktoren) oder eine Veränderung der äußeren Bedingungen beeinflusst. Diese Induktoren I₁ bzw I₂ können beispielsweise Biomoleküle sein, welche der Wirt synthetisiert, oder natürliche oder künstliche Moleküle, welche von außen zugegeben werden. Als Veränderung der äußeren Bedingungen kommt insbesondere auch eine Temperaturveränderung in Betracht.

Besonders bevorzugt ist I₁ Induktor für P₁, nicht jedoch für P₂, und/oder I₂ ist Induktor für P₂, nicht jedoch für P₁.

In einer bevorzugten Ausführungsform umfasst der Promotor P₁ und/oder der Promotor P₂ neben der Bindungsstelle für die RNA-Polymerase wenigstens eine außerhalb dieser Bindungsstelle liegende Verstärker-Sequenz (Enhancer-Sequenz) und/oder wenigstens eine Operator-Sequenz.

Enhancer sind typischerweise in der *3'-untranslated region* der zur exprimierenden Sequenz lokalisiert. Solche Enhancer-Sequenzen können prokaryotischen oder eukaryotischen Ursprungs sein. Es kann sich um Varianten dieser Sequenzen oder um synthetische Enhancer-Sequenzen handeln.

In einer Ausführungsform ist die Enhancer-Sequenz die Wild-Typ-Enhancer-Sequenz des ausgewählten Promotors.

Vorzugsweise umfassen P₁ und P₂ jeweils unabhängig voneinander höchstens 1000 bp, bevorzugter höchstens 900 bp und besonders bevorzugt höchstens 800 bp.

Einen Einfluss auf die Expressionsrate in prokaryotischen Wirten hat auch die Anwesenheit/ Ausführungsform der Shine-Dalgarno-Sequenz. Die Konsensus-Sequenz der Shine-Dalgarno-Sequenz in *E*. *coli* lautet AGGAGG. In einer bevorzugten Ausführungsform wird im Zusammenhang mit dem Promotor P₁ eine Shine-Dalgarno-Sequenz verwendet, die an mindestens 4 Nukleotiden, bevorzugter mindestens 5 Nukleotiden, noch bevorzugter 6 Nukleotiden, am bevorzugtesten gänzlich mit der Konsensussequenz übereinstimmt.

In einer bevorzugten Ausführungsform wird im Zusammenhang mit dem Promotor P₂ eine Shine-Dalgarno-Sequenz verwendet, die an mindestens 4 Nukleotiden, bevorzugter mindestens 5 Nukleotiden, noch bevorzugter 6 Nukleotiden, am bevorzugtesten gänzlich mit der Konsensussequenz übereinstimmt.

Ähnlichen Einfluss auf die Expressionsrate in eukaryotischen Wirten die Kozak-Sequenz.

Die Kozak-Sequenz für Säugetiere zum Beispiel hat die Konsensus-Sequenz (GCC)GCCR-CCAUGG (<SEQ.ID.NO: 3>), wobei R ein Purin ist, welches 3 bp stromaufwärts des Startcodons AUG liegt und wobei stromabwärts vom Startcodon ein Guanin liegt und die Kozak-Sequenz von Hefen hat zum Beispiel die Konsensus-Sequenz von (A/U)A(A/C)AA(A/C)AUGUC(U/C) (<SEQ.ID.NO: 4>).

In einer bevorzugten Ausführungsform wird im Zusammenhang mit dem Promotor P₁ in einem eukaryotischen Wirt die Konsensussequenz verwendet.

In einer bevorzugten Ausführungsform wird im Zusammenhang mit dem Promotor P₂ in einem eukaryotischen Wirt die Konsensussequenz verwendet.

In einer anderen bevorzugten Ausführungsform sind auf dem erfindungsgemäßen leeren Expressionsvektor weder eine Shine-Dalgarno-Sequenz noch eine Kozak-Sequenz auf der Insertionssequenz in beiden Leserichtungen angeordnet. Diese bevorzugte Ausführungsform bezieht sich auf den Expressionsvektor im ursprünglichen Zustand, d.h. in demjenigen Zustand, in dem noch keine zu exprimierende oder sonstige DNA in die Insertionssequenz (z.B. den Polylinker) kloniert wurde. Ein solcher Vektor ist dem Fachmann auch als "leerer Vektor" bekannt. In dieser Ausführungsform des erfindungsgemäßen Experssionsvektors umfasst dann bevorzugt die in die Insertionssequenz zu klonierende Sequenz eine Shine-Dalgarno-Sequenz bzw. eine Kozak-Sequenz.

Die *in vivo* Promotorstärke ist durch die RNA-Syntheserate, die durch eine einzelne Promotorsequenz ausgelöst wird, definiert und führt zu einem entsprechenden Anteil des gewünschten Zielproteins am Gesamtproteingehalt des Wirtsorganismus. Die verwendeten Promotoren führen zu einem Gehalt eines exprimierten Zielproteins in Bezug auf den Gesamtproteingehalt von bevorzugt > 1%, bevorzugter > 5 %, noch bevorzugter > 10 %, am bevorzugtesten > 25 % insbesondere > 50 %.

Die beiden Promotoren P₁ und P₂ laufen erfindungsgemäß aufeinander zu, d.h. sie sind konvergent, *face-to-face.* Aufeinander zulaufende Promotoren werden dadurch realisiert, dass der Promotor P₁ auf dem einen DNA-Stang und der Promotor P₂ auf dem dazu komplementären DNA-Stang des Expressionsvektors angeordnet ist. Mit anderen Worten sind erfindungsgemäß der Promotor P₁ und die zum Promotor P₂ komplementäre Sequenz auf dem einen DNA-Strang und der Promotor P₂ und die zum Promotor P₁ komplementäre Sequenz auf dem komplementären DNA-Stang des Expressionsvektors angeordnet.

Konvergente Promotoren sind von bidirektionalen Promotoren zu unterscheiden, auch wenn beide Begriffe in der Literatur gelegentlich synonym verwendet werden.

Im eigentlichen Sinn bezeichnet ein bidirektionaler Promotor eine Promotorregion bzw. zwei *back-to-back* klonierte Promotoren, deren Leserichtung voneinander weg weist, und mit denen zwei die Promoterregion flankierende offene Leserraster abgelesen werden. Solche Promotoren sind weit verbreitet, da sie bei der Coexpression eines im stöchiometrischen Verhältnis zum Zielgen vorliegenden Reportergens, insbesondere in Zellkulturen, genutzt werden können. In diesem Zusammenhang kann beispielsweise verwiesen werden auf Sammarco et al., Anal. Biochem. 2005, 346(2), 210-216; Baron et al. Nucleic Acids Res. 1995, 23(17), 3605-6; und EP-A 1 616 012.

Im Unterschied dazu handelt es sich bei konvergenten Promotoren, wie bei den erfindungsgemäßen Promotoren P₁ und P₂, um zwei *face*-*to*-*face* klonierte Promotoren, deren Leserichtung aufeinander zuweist. Infolge der zirkulären Struktur von Plasmiden und anderen zirkulär vorliegenden Expressionsvektoren können auch bidirektionale Promotoren in gewisser Weise face-to-face orientiert sein, allerdings nicht in Bezug auf die Insertionssequenz, welche erfindungsgemäß bevorzugt zwischen den beiden Promotoren P₁ und P₂ jeweils stromabwärts angeordnet ist, so dass die beiden Promotoren P₁ und P₂ die Insertionssequenz auf beiden Seiten flankieren. Auf diese Weise kann über die Promotoren die Expression von DNA-Sequenzen gesteuert werden, welche zuvor in den Bereich der Insertionssequenz kloniert wurden, und zwar in beiden Leserichtungen.

Erfindungsgemäß bevorzugt ist daher zwischen P₁ und P₂ eine Insertionssequenz angeordnet, und zwar jeweils stromabwärts, so dass die Expression einer in die Insertionssequenz klonierten DNA-Sequenz unter die Kontrolle von P₁ und P₂ gestellt wird. Mit anderen Worten laufen P₁ und P₂ sowohl aufeinander, als auch auf die Insertionssequenz zu.

Solcherlei Insertionssequenzen sind dem Fachmann bekannt. Bevorzugt handelt es sich bei einer solchen Insertionssequenz um einen Polylinker.

Zum Zwecke der Beschreibung versteht man unter einem Polylinker (dem Fachmann auch bekannt als *multiple cloning site* (MCS)) einen DNA-Abschnitt in einem Vektor, dessen Sequenz in kurzer Abfolge hintereinander verschiedene Schnittstellen für Restriktionsendonukleasen enthält. Dieser ermöglicht ein flexibles Klonieren, da aus den verschiedenen Restriktionsschnittstellen die jeweils am besten passende ausgewählt und verwendet werden kann. Die Schnittstellen sind dabei einzigartig (*unique*) auf dem Vektor.

In einer bevorzugten Ausführungsform umfasst der Polylinker wenigstens 1, bevorzugter wenigstens 2 oder wenigstens 3, noch bevorzugter wenigstens 4 oder wenigstens 5, am bevorzugtesten wenigstens 6 oder wenigstens 7 und insbesondere wenigstens 8 oder wenigstens 9 Erkennungssequenzen für Restriktionsendonucleasen, welche ggf. überlappen. In diesem Zusammenhang werden unter Restriktionsendonukleasen vorzugsweise solche Restriktionsendonukleasen des Typs I, II oder III verstanden, welche in der REBASE-Datenbank (http://rebase.neb.com/rebase) aufgeführt sind. Weiterhin wird in diesem Zusammenhang unter Erkennungssequenzen für Restriktionsendonucleasen vorzugsweise Penta-, Hexa-, Hepta- oder Octamere vorzugsweise einer doppelsträngigen DNA-Sequenz verstanden. Vorzugsweise sind die Hexa- oder Octamere palindromisch, d.h dass sie auf beiden Strängen in einer Richtung (zum Beispiel 5'-3') die gleiche Basenabfolge zeigen, z.B. GAATTC oder GCGGCCGC. In einer weitere bevorzugten Ausführungsform sind diese Erkennungssequenzen unterbrochen, dass heißt zwischen Teilen der fixierten Erkennungssequenzen liegen frei wählbare Sequenzen, z.B. CACNNNNGTG oder GCNNGC.

In einer anderen bevorzugten Ausführungsform umfasst der Polylinker einen Sequenzabschnitt von höchstens 20 bp, vorzugsweise von höchstens 15 bp, auf dem mindestens 1 oder mindestens 2, bevorzugter mindestens 3 oder mindestens 4, noch bevorzugter mindestens 5 oder mindestens 6, am bevorzugtesten mindestens 7 oder mindestens 8, und insbesondere mindestens 9 oder mindestens 10 Schnittstellen Restriktionsendonukleasen liegen, welche ggf. überlappen können. In diesem Zusammenhang werden unter Restriktionsendonukleasen vorzugsweise solche Restriktionsendonukleasen des Typs I, II oder III verstanden, welche in der REBASE-Datenbank (http://rebase.neb.com/rebase) aufgeführt sind.

Neben Restriktionsendonukleasen kommen grundsätzlich auch Homing-Endonukleasen in Betracht.

In einer bevorzugten Ausführungsform ist zwischen dem letzten bp des Promotors P₁ und dem letzten bp des Promotors P₂ in face-to-face Anordnung eine Insertionssequenz angeordnet, welche höchstens 500 bp, bevorzugter höchstens 200 bp, noch bevorzugter höchstens 100 bp, noch bevorzugter höchstens 50 bp, am bevorzugtesten höchstens 20 bp und insbesondere höchstens 6 bp umfasst. In diesem Zusammenhand bezieht sich der Ausdruck "letztes bp" auf die Leserichtung der RNA-Polymerase. Diese bevorzugte Ausführungsform bezieht sich auf den Expressionsvektor im ursprünglichen Zustand, d.h. in demjenigen Zustand, in dem noch keine zu exprimierende oder sonstige DNA in die Insertionssequenz (z.B. den Polylinker) kloniert wurde (leerer Vektor).

In einer besonders bevorzugten Ausführungsform liegen auf der Insertionssequenz höchstens 100, bevorzugter höchstens 50, bevorzugter höchstens 20, bevorzugter höchstens 10 Schnittstellen, bevorzugter höchstens 5 Schnittstellen und besonders bevorzugt höchstens 1 Schnittstelle von Restriktionsendonukleasen, welche vorzugsweise eine Erkennungssequenz zwischen 4 und 10 b aufweisen und überhängende oder glatte Enden erzeugen. Besonders vorzugsweise sind die Restriktionsendonucleasen ausgewählt aus der Gruppe bestehend aus Aanl (Psil), Aarl, Aasl (Drdl), Aatll, Acc65I (Kpnl), Adel (DraIII), Ajil (BmgBI), Ajul, AlfI, Alol, Alul, Alw21I (BsiHKAI), Alw26I (BsmAI), Alw44I (ApaLI), Apal, BamHI, BauI (BssSI), Bell, Bcnl (Ncil), Bcul (Spel), Bdal, Bfil (BmrI), Bfml (Sfcl), Bful (BciVI), BgII, BglII, Bme1390I ScrFI), BoxI (PshAI), Bpil (BbsI), Bpll, Bpu10I, Bpu1102I (BlpI), BseDI (BsaJI), BseGI (Fokl), BseJI (BsaBI), BseLI (BsII), BseMI (BsrDI), BseMII (BspCNI), BseNI (BsrI), BseSI Bme1580I), BseXI (Bbvl), Bsh1236I (BstUI), Bsh1285I (BsiEI), BshNI (BanI), BshTI (AgeI), Bsp68I (Nrul), Bsp119I (BstBI), Bsp120I (PspOMI), Bsp143I (Sau3AI), Bsp1407I (BsrGI), BspLI (NlaIV), BspO1 (Bmtl), BspPI (Alwl), BspTI (AflII), Bst1107I (BstZ17I), BstXI, Bsu15I ClaI), BsuRI (HaeIII), Bvel (BspMI), Cail (AlwNI), CfrI (Eael), Cfr9I (Xmal), Cfr10I (BsrFI), Cfr13I (Sau96I), Cfr42I (Sacll), Cpol (Rsrll), Csel (Hgal), Csp6I (CviQI), Dpnl, DraI, Eam1104I (Earl), Eam1105I (Ahdl), Ecl136II (EcolCRI), Eco24I (BanII), Eco31I (Bsal), Eco32I (EcoRV), Eco47I (Avall), Eco47III (Afel), Eco52I (Eagl), Eco57I (Acul), Eco57MI, Eco72I (Pmll), Eco81I (Bsu36I), Eco88I (Aval), Eco91I (BstEII), Eco105I (SnaBI), Eco130I (Styl), Eco147I (Stul), EcoO109I (Drall), EcoRI, EcoRII, EheI (Narl), Esp3I (BsmBI), FaqI (BsmFI), FspAI, FspBI (Bfal), Gsul (Bpml), Hhal, Hin1I (Acyl), Hin1II (NlaIII), Hin4I, Hin6I (HinPII), HincII (HindII), HindIII, Hinfl, HpaII, HphI, Hpy8I (MjaIV), HpyF3I (DdeI), HpyF10VI (Mwol), KpnI, Kpn2I (BspEI), KspAI (Hpal), Lgul (Sapl), Lsp1109I (Bbvl), Lwel (SfaNI), MauBI Mbil (BsrBI), Mbol, Mboll, MlsI (Mscl), Mlul, Mnll, Mph1103I (Nsil), Mrel (Sse232I), Mspl (Hpall), Mssl (PmeI), MunI (Mfel), MvaI (BstNI), Mva1269I (Bsml), Ncol, Ndel, NheI, NmuCI (Tsp45I), Notl, Nsbl (Fspl), Olil (AleI), PaeI (Sphl), PagI (BspHI), PasI, Paul (BssHII), Pdil (NaeI), Pdml (Xmnl), Pfel (Tfil), Pfl23II (BsiWI), Pfol, Ppil, Ppu21I (BsaAI), Pscl (Pcil), Psp5II (PpuMI), Psp1406I (AcII), Pstl, PsuI (BstYI), Psyl (Tth111I), PvuI, Pvull, Rsal, Rsel (MslI), Sacl, SalI, Satl (Fnu4HI), Scal, Schl (PleI), Sdal (Sbfl), Sdul (Bsp1286I), SfaAI (AsiSI), Sfil, SgrDI, Sgsl (AscI), Smal, Smil (Swal), SmoI (Smll), Smul (Faul), Ssil (Acil), Sspl, Taal (HpyCH4III), TaiI (Maell), Taql, Tasl (Tsp509I), TatI, TauI, Tru1I (Msel), TscAI (TspRI), Tsol, Tstl, Van91I (PflMI), Vspl (AseI), Xagl (EcoNI), Xapl (ApoI), Xbal, Xcel (Nspl), Xhol, XmaJI (Avrll) und Xmil (AccI).

Besonders bevorzugt umfasst die Insertionssequenz höchstens 50 bp und weist mindestens 6 Schnittstellen für Restriktionsendonukleasen auf.

Um eine Translation in allen drei *reading frames* zu gewährleisten, ist in einer bevorzugten erfindungsgemäßen Ausführungsform auch ein System von Expressionsvektoren umfasst, indem die ganze Sequenz oder Teile der Sequenz des Polylinkers jeweils um ein Nukleotid im Hinblick auf die restliche Vektorsequenz verschoben sind. Zur Illustration einer solchen Lehre wird auf die Publikationen von Charnay et al. (1978) Nucl. Acid Res. 5: 4479 und Villa-Komaroff (1978) Proc. Natl. Acad. Sci. 75, 3727 verwiesen.

In einer anderen bevorzugten Ausführungsform umfasst der erfindungsgemäße leere Expressionsvektor keinen Translationsstart, d.h. auch kein Startcodon ATG oder GTG innerhalb der Insertionssequenz in beiden Leserichtungen. In dieser bevorzugten Ausführungsform enthält dann bevorzugt die in die Insertionssequenz zu klonierende Sequenz einen solchen Translationsstart inklusive eines Startcodons.

In einer bevorzugten Ausführungsform liegt auf der Insertionssequenz in beiden Leserichtungen keine Ribosomenbindungsstelle. Dadurch wird gewährleistet, dass vom leeren Vektor von beiden Promotoren keine Translation der entstehenden mRNA initiiert werden kann.

Besonders bevorzugt enthält der erfindungsgemäße leere Expressionsvektor weder Ribosomenbindungsstellen noch Startcodons in der Insertionssequenz in beiden Leserichtungen.

In einer besonders bevorzugten Ausführungsform liegt auf der Insertionsssequenz (noch) kein Gen, z.B. für eine bestimmte Antibiotikaresistenz, so dass der leere Expressionsvektor zwischen P₁ und P₂ nur die Insertionssequenz als solche enthält. Auf diese Weise wird gewährleistet, dass sich beide Promotoren funktionell auf die Insertionssequenz beziehen, d.h. auf beide DNA-Stränge der Insertionssequenz, so dass eine Klonierung in die Insertionssequenz ungerichtet erfolgen kann. In diesem Zusammenhang bedeutet "ungerichtet", dass es erfindungsgemäß letztlich nicht darauf ankommt, in welchen der beiden DNA-Stränge des Plasmids eine bestimmte Sequenz insertiert wird, da sich beide Promotoren funktionell auf die Insertionssequenz beziehen, wird die insertierte Sequenz zwangsläufig entweder unter die Kontrolle von P₁ oder unter die Kontrolle von P₂ gestellt. Eine Expression der insertierten Sequenz wird auf diese Weise in jedem Fall gewährleistet.

Wäre hingegen im leeren Experssionsvektor bereits ein Gen unter die Kontrolle von z.B. P₁ gestellt, z.B. ein Gen für eine bestimmte Antibiotikaresistenz, so wäre eine ungerichtete Klonierung nicht möglich (oder zumindest mit Nachteilen verbunden), da eine (weitere) Insertion stromabwärts von P₁ stets eine Kopplung der Expression der insertrierten Sequenz mit dem bereits vorhandenen Gen zu Folge hätte. Für den Fall, dass auf das Gen für die Antibiotikaresistenz ein Terminator folgte, würde die inserierte Fremd-DNA, welche nach dem Gen eingefügt würde, nur eingeschränkt oder gar nicht unter der Kontrolle des betreffenden Promotors stehen, und der erfindungsgemäße Vorteil der zwei auf die gleiche Insertionssequenz gerichteten Promotoren wäre dahin.

Eine Entkopplung der Expression der insertierten Sequenz von dem unter der Kontrolle von P₁ befindlichen Gen würde eine hingegen gerichtete Klonierung in die Insertionssequenz stromabwärts von P₂ erforderlich machen, d.h. gezielt in den anderen DNA-Strang. Gerichtete Klonierungen machen jedoch eine entsprechende 5'-3'-Ausrichtung der zu insertierenden Sequenz erforderlich, so dass mit Hilfe eines solchen Expressionsvektors letztlich nur noch 50% einer DNA-Varianten-Bibliothek durchmustert werden könnten.

In einer alternativen Ausführungsform kann im erfindungsgemäßen Expressionsvektor als Insertionssequenz anstatt oder zusätzlich eines Polylinkers auch eine Sequenz vorliegen, die eine Integration von DNA-Sequenzen mittels Rekombination ermöglicht.

Verfahren zur Integration von DNA-Sequenzen in einen Vektor, vorzugsweise Expressionsvektor, sind dem Fachmann bekannt. Beispielsweise basiert ein solches Verfahren auf der Rekombination über att-sites, wie es beispielsweise in den GATEWAY-Vektoren der Firma Invitrogen (Carlsbad, CA, USA). Ein anderes Verfahren ist beschrieben in Muyrers J.P.P, Zhang Y, and Stewart A.F. (2001) "Recombinogenic engineering- new options for cloning and manipulating DNA" TIBS 26: 325-331. Die zu klonierende DNA eine (Meta-)Genombank müsste dazu dann mit entsprechenden Linkern vorbehandelt werden. Methoden zum Anbringen von Linkern an DNA sind dem Fachmann bekannt.

In einer bevorzugten Ausführungsform ist hinter dem letzten bp von P₁ und/oder hinter dem letzten bp von P₂, jedoch vor dem Polylinker, eine Sekretionssequenz angeordnet, welche dazu dient, dass der Wirt nach der Expression das exprimierte Peptid bzw. Protein sekretiert. Hierzu ist es notwendig, dass zwischen der Sekretionssequenz und dem Polylinker kein Stopp-Codon vorliegt. Es wird dann in den klonierten DNA-Sequenzen vorzugsweise nach solchen Sequenzen gesucht, welche durch die Klonierung ein Fusionsprotein aus Signalpeptid und codierten Protein ergeben. Geeignete Sekretionssequenzen sind biologisch definiert und dem Fachmann bekannt.

In einer weiteren bevorzugten Ausführungsform umfasst die Insertionsseqeunz neben den Polylinker und/oder DNA-Sequenzen zur Rekombination auch eine sogenannte Suizid-Sequenz. Suizid-Sequenzen sind solche Sequenzen, die zum Absterben bestimmter Wirte führen. So codiert die Suizid-Sequenz zum Beispiel für eine Restriktionsendonuclease (z.B. EcoRI), welche durch Verdauung der genomischen DNA zum Absterben von solchen Wirten führt, die nicht eine dazugehörige Methyltransferase (z.B. EcoMI) codieren, welche die eigene DNA schützt. Die Schnittstellen des Polylinkers sind in diesem Fall innerhalb der Suizid-Sequenz angeordnet. Werden nun in den Polylinker zusätzliche DNA-Sequenzen kloniert, so wird dadurch das Suizid-Gen unterbrochen und inaktiv. Dadurch wird verhindert, dass bei der Klonierung der DNA und anschließender Transformation der Vektoren in geeignete Wirte sogenannte Religanden, d.h. ohne zusätzliche DNA wieder religierte Vektoren, entstehen. Vorzugsweise wird der erfindungsgemäße Expressionsvektor in diesem Falle in einem Wirt produziert, der die entsprechende Schutz-Methyltransferase exprimiert, während die Erstellung der Banken dann in einem Wirt erfolgt, der die Schutz-Methyltransferase nicht codiert. Verschiedenste weitere Suizid-Systeme sind dem Fachmann bekannt. Beispielsweise sei auf das pJET-System der Firma Fermentas (Vilnius, Litauen); Quandt J und Hynes MF (1993) "Versatile suicide vectors which allow direct selection for gene replacement in gram-negative bacteria", Gene 127,15-21; Ortiz-Martin et al., (2006) "Suicide vectors for antibiotic marker exchange and rapid generation of multiple knockout mutants by allelic exchange in Gram-negative bacteria", J Microbiol Methods. 67, 395-407; Schlieper et al., (1998) "A Positive Selection Vector for Cloning of Long Polymerase Chain Reaction Fragments Based on a Lethal Mutant of the crp Gene of Escherichia coli", Anal. Biochem. 257, 203-209 oder Bej et al., (1988) "Model suicide vector for containment of genetically engineered microorganisms.", Appl Environ Microbiol. 54, 2472-7 verwiesen.

Konvergente Promotoren sind im Stand der Technik bekannt. So liegen bei einigen kommerziellen Klonierungsplasmiden zwei konvergente Promotoren beidseitig des Polylinkers (*multiple cloning site,* MCS), z.B. T7 und SP6-Promotor in pDrive (Merck, Darmstadt). Bei diesen Klonierungsplasmiden handelt es sich jedoch um keine Expressionsplasmide, da sie nicht zur funktionellen Expression der klonierten Gene *in vivo* dienen, sondern lediglich zur Generierung von RNA durch *in vitro* Transkription, z.B. für Northern Blots, und als häufig verwendete Primersites für das Sequenzieren. Ferner sind die konvergenten Promotoren auf diesen Klonierungsvektoren nicht unabhängig induzierbar. Konvergente Promotoren sind ferner beschrieben für Plasmide, mit denen simultan sense und *antisense* RNA produziert werden soll, um siRNA und dsRNA für das *gene silencing* in Eukaryoten zu gewinnen (vgl. z.B. Waterhouse et al., Plant Biology, 1998, 95, 13959-64; Zheng et al., PNAS, 2004, 101, 135-40. Konvergente Promotoren kommen auch natürlich in Bakterien vor, z. B. in *Bacillus,* wo zwei Promotoren das Ablesen zweier unterschiedliche Genprodukte auf dem *sense* und *antisense* Strang des gleichen DNA Abschnitts bewirken (Wang et al., J. Bacteriol., 1999, 181, 353-6).

Die Verwendung eines Vektors mit zwei konvergenten Promotoren zum Durchmustern einer (Meta)genom-Bibliothek ist ebenfalls in der Literatur beschrieben (vgl. Lämmle et al., Journal of Biotechnology, 2007, 127, 575-92). Es handelt sich hierbei um den Vektor pJOE930 (Altenbuchner et al., Methods Enzymol., 1992, 216, 457-66), welcher zwei konvergente, vergleichsweise schwache lac-Promotoren trägt und zur Klonierung und IPTG-induzierten Expression von metagenomischer DNA eingesetzt werden kann. Die palindromische Sequenz der beiden lac-Promotoren und der von ihnen umschlossenen MCS bewirken eine Instabilität des Leervektors in *E*. *coli.* Darüber hinaus sind die beiden Promotoren infolge ihrer Gleichartigkeit nicht getrennt induzierbar.

Es wurde überraschend gefunden, dass getrennt induzierbare konvergente Promotoren Vorteile gegenüber nicht getrennt induzierbaren konvergenten Promotoren aufweisen.

Zum Zwecke der Beschreibung bedeutet eine getrennte Induzierbarkeit der Promotoren P₁ und P₂, dass der Promotor P₁ durch geeignete Maßnahmen selektiv induziert werden kann, ohne dass gleichzeitig auch der Promotor P₂ in signifikantem Maß induziert wird, und umgekehrt. Vorzugsweise wird bei selektiver Induktion des Promotors P₁ der Promotor P₂ um höchstens 10% seiner maximalen Induzierbarkeit induziert, bevorzugter höchstens 1%, noch bevorzugter höchstens 0,5%, am bevorzugtesten höchstens 0,2% und insbesondere höchstens 0,1%, und umgekehrt. Eine getrennte Induzierbarkeit der Promotoren kann im einfachsten Fall dadurch erreicht werden, dass Promotoren P₁ und P₂ verwendet werden, welche mit unterschiedlichen Modulatoren (Repressoren, Aktivatoren) zusammenwirken.

Der erfindungsgemäße leere Expressionsvektor weist ohne Insertionssequenz insgesamt höchstens 3000 bp auf, d.h. die Gesamtsequenz des Expressionsvektors einschließlich P₁ und P₂ jedoch abzüglich Insertionssequenz umfasst höchstens 3000 bp.

In einer bevorzugten Ausführungsform umfasst der erfindungsgemäße leere Expressionsvektor nach Öffnen in der Insertionssequenz bzw. nach Herausschneiden von nicht benötigten Teilen der Insertionssequenz insgesamt höchstens 3000 bp, bevorzugter höchstens 2900 bp, bevorzugter höchstens 2800 bp, bevorzugter höchstens 2700 bp, noch bevorzugter höchstens 2600 bp, am bevorzugtesten höchstens 2550 bp und insbesondere höchstens 2500 bp.

In einer anderen bevorzugten Ausführungsform umfasst der erfindungsgemäße leere Expressionsvektor als solcher insgesamt höchstens 3000 bp, bevorzugter höchstens 2900 bp, bevorzugter höchstens 2800 bp, bevorzugter höchstens 2700 bp, noch bevorzugter höchstens 2600 bp, am bevorzugtesten höchstens 2550 bp und insbesondere höchstens 2500 bp.

In einer weiteren bevorzugten Ausführungsform umfasst der erfindungsgemäße leere Expressionsvektor ohne Insertionssequenz insgesamt höchstens 2900 bp, bevorzugter höchstens 2800 bp, bevorzugter höchstens 2700 bp, noch bevorzugter höchstens 2600 bp, am bevorzugtesten höchstens 2550 bp und insbesondere höchstens 2500 bp.

Bevorzugt kodiert der erfindungsgemäße Expressionsvektor nicht für einen Regulator von P₁ und/oder nicht für einen Regulator von P₂.

In einer bevorzugten Ausführungsform des erfindungsgemäßen Expressionsvektors ist P₁ ein T7 Promotor. Der T7 Promotor ist dem Fachmann bekannt. In diesem Zusammenhang kann beispielsweise vollumfänglich verwiesen werden auf Studier und Moffatt (1986) "Use of bacteriophage T7 RNA polymerase to direct selective high-level expression of cloned genes" J Mol Biol 189, 113-130. Der Begriff "T7-Promotor" bezeichnet im Sinne der vorliegenden Erfindung einen Promotor der von der T7-RNA-Polymerase als Transkriptionsstart erkannt wird und der um mindestens eine lacO Operatorsequenz erweitert wurde. *LacI* ist dann der Repressor des T7-Promotors.

In einer bevorzugten Ausführungsform des erfindungsgemäßen Expressionsvektors ist P₂ ein Promotor, welcher durch Arabinose (I₂) reguliert wird, insbesondere der *ara*-Promotor.

In einer bevorzugten Ausführungsform handelt es sich hierbei um einen *ara*-Promotor aus gram-negativen Bakterien, vorzugsweise *E*. *coli.* In diesem Fall kodiert der erfindungsgemäße Expressionsvektor bevorzugt nicht für den Regulator AraC des *ara-*Promotors.

Der *ara*-Promotor ist dem Fachmann bekannt. Das Arabinose-Operon besteht aus einer regulierbaren Promotorregion (*ara-*Promotor), sowie drei Strukturgenen (*araB, ara A* und *araD*), welche Proteine zum Abbau von L-Arabinose kodieren. *AraC* wird konstitutiv exprimiert. Das Genprodukt dient als Repressor. Er bindet an den Promotor und verhindert so die Transkription der Gene *araB, araA* und *araD.* Liegt Arabinose vor, bindet diese an AraC. Dadurch, dass Arabinose gebunden wird, verändert AraC seine Form, bindet an andere DNA-Sequenzen und wird so zum Aktivator. Deshalb kann sich jetzt die RNA-Polymerase an den Promotor anlagern und die Transkription der Strukturgene beginnt. Wenn die Arabinose vollständig abgebaut ist, ändert AraC wieder die Konformation und stoppt wieder die Transkription. Bezüglich weiterer Einzelheiten kann beispielsweise vollumfänglich verwiesen werden auf Schleif R. (2000) Regulation of the L-arabinose operon of Escherichia coli. Trends Genet. 16, 559-65.

In einer weiteren bevorzugten Ausführungsform ist der erfindungsgemäße Expressionsvektor dadurch gekennzeichnet, dass er für jeweils mindestens einen Terminator T₁ bzw. T₂ in entsprechender Leserichtung der Promotoren P₁ bzw. P₂ kodiert.

In einer bevorzugten Ausführungsform hiervon liegt auf dem Expressionsvektor folgende Anordnung von P₁, P₂, T₁, T₂ und der Insertionssequenz vor: T₂ (antisense) - P₁ (sense) - Insertionssequenz (sense/antisense) - P₂ (antisense) - T₁ (sense).

Besonders bevorzugt ist T₁ ein T7-Terminator.

Besonders bevorzugt ist T₂ ein Terminator für die Wirts-RNA-Polymerase.

In einer bevorzugten Ausführungsform ist der Terminator für den T7-Promotor der T7-Terminator und der Terminator für den *ara*-Promotor eine Terminatorsequenz für die *E*. *coli* RNA-Polymerase. In einer besonders bevorzugten Ausführungsform wird für den *ara-*Promotor kein eigenständiger Terminator kloniert, sondern der Terminator des in antisense klonierten stromaufwärts liegenden Genes des Expressionsvektors genutzt.

Im Rahmen der vorliegenden Erfindung bezeichnet "Tᵢ wahlweise T₁ oder T₂.

In einer weiteren besonders bevorzugten Ausführungsform ist der Expressionsvektor dadurch gekennzeichnet, dass zwischen Pᵢ und seinem Terminator Tᵢ in Leserichtung von Pᵢ aber nach dem zweiten Promotor Pⱼ ein zusätzliches Gen liegt.

Weiterhin umfasst der erfindungsgemäße Expressionsvektor eine Selektionsmarkersequenz, welche geeignet ist, Wirte, die den Expressionsvektor enthalten, von Wirten zu unterscheiden, die den Expressionsvektor nicht enthalten.

Dieses kann beispielsweise dadurch erreicht werden, dass die Selektionsmarkersequenz dem Wirt eine Antibiotikaresistenz verleiht, so dass er auf Nährmedien überlebensfähig ist, auf denen andere Wirte, welche den Expressionsvektor nicht enthalten, absterben. Geeignete Sequenzen, welche die Antibiotikaresistenz verleihen, sind dem Fachmann bekannt. Bevorzugt ist das Antibiotikum, gegen welches durch die Selektionsmarkersequenz Resistenz verliehen wird, ausgewählt aus der Gruppe bestehend aus Ampicillin, Tetracyclin, Kanamycin, Chloramphenicol, Spectinomycin, Hygromycin, Sulphonamid, Trimethoprim, Bleomycin/Phleomycin, Zeocin^{™}, Gentamycin und Blasticidin.

Alternativ können auxotrophe Wirte (Negativmutanten) verwendet werden, welche zum Überleben auf einen bestimmten Nährstoff angewiesen sind (Aminosäure, Kohlenhydrat, etc.), welchen sie selbst nicht synthetisieren können. Auf einem Nährmedium, welches diesen Nährstoff nicht bereitstellt, sind diese Wirte dann nicht überlebensfähig. In diesem Fall verleiht die Selektionsmarkersequenz auf dem erfindungsgemäßen Expressionsvektor dem Wirt die Fähigkeit, diesen Nährstoff zu synthetisieren, so dass eine Überlebensfähigkeit auf dem defizienten Nährmedium durch den Expressionsvektor induziert wird. Geeignete Selektionsmarkersequenzen sind dem Fachmann bekannt.

Im Falle von Hefe-Zellen können als Marker solche verwendet werden, die es auxotrophen Hefestämmen ermöglichen ohne zusätzliches Uracil, Tryptohan, Histidin, Leucin oder Lysin im Medium zu wachsen.

Im Falle von Säuger-Zellen können als Marker beispielsweise Sequenzen verwendet werden, welche die Aktivität der DHFR, der Cytosin-Deaminase, der Hygromycin-ßphosphotransferase (HPH), der Puromycin-N-acetyl transferase (PAC), der Thymidine kinase (TK) und der Xanthine-guanine phosphoribosyltransferase (XGPRT) kodieren.

Alternativ können Sequenzen verwendet werden, die einen Counterselektions-Marker kodieren, wie beispielsweise das *sacB* Gen von *B. subtilis* oder das F-Plasmid *ccdB-Gen* oder Colicin-Release-Gene wie das *kil*-Gen für ColicinE1.

Ein anderes Beispiel ist die Verwendung eines Fragments des Phagen Mu wie beschrieben in Schumann (1979) Mol. Gen. Genet. 174, 221-4. Andere Bespiele solcher Marker sind beschrieben in Roberts et al. (1980) Gene 12, 123-7; Dean (1981) Gene 15, 99-102, Hennecke et al. (1982) Gene 19, 231-4 oder Hashimoto-Gotoh et al. (1986) Gene 41, 125-8.

Zusätzlich können Sequenzen verwendet werden, die eine Selektion auf Basis der blau/ weiß-Färbung nach IPTG/X-GAL-Zugabe erfolgt.

Zusätzlich können Sequenzen in den Bereich zwischen den Promotoren P₁ und P₂ inseriert werden, die ein Screening mittels PCR ermöglichen.

In einer Ausführungsform können Expressionsvektoren verwendet werden, die eine Coexpression der klonierten Sequenz mit einem detektierbaren Marker erlauben. Bei einem solchen detektierbaren Marker kann es sich beispielsweise um einen Tag wie einen His-tag, einen Poly-His-tag, einrn MAT-tag, einen Streptavidin-tag, einen Streptavidine-binding-tag, einen GST-tag, einen Antikörper-Bindungs-tag, einen Myc-tag, einen Swa11-Epitop oder einen FLAG-tag handeln. In einer Ausführungsform kann es sich auch um fluoreszierende Tags handeln wie einen GFP-tag, einen BFP-tag oder einen RFP-tag.

In einer bevorzugten Ausführungsform weist der erfindungsgemäße Expressionsvektor wenigstens 70%, bevorzugter wenigstens 80%, noch bevorzugter wenigstens 85%, am bevorzugtesten wenigstens 90% und insbesondere wenigstens 95% Homologie zu <SEQ ID NO: 1> auf. Die Homologie wird hierbei bevorzugt mit Hilfe des Algorithmus nach Smith & Waterman (J Mol Biol., 1981, 147(1), 195-7) ermittelt, unter Verwendung der BLOSUM62-Matrix und Werten von 11.0 für die Eröffnung einer Lücke, bzw. 1.0 für die Erweiterung einer Lücke.

Ein weiterer Aspekt der Erfindung betrifft ein Expressionssystem umfassend den vorstehend beschriebenen Expressionsvektor und separat vorliegende regulatorische Sequenzen, welche für einen Regulator R₁ von P₁ und/oder für einen Regulator R₂ von P₂ kodieren. In diesem Zusammenhang bedeutet "separat", dass die regulatorischen Sequenzen nicht auf dem erfindungsgemäßen Expressionsvektor, bzw. einem oder mehreren in das Wirtschromosom integrierten Teilstücken, liegen. Vorzugsweise liegen die regulatorischen Sequenzen auf einem Vektor (Regulatorvektor), welcher für einen Regulator R₁ von P₁ und/oder für einen Regulator R₂ von P₂ kodiert. Vorzugsweise ist R₁ Lacl und/oder R₂ AraC.

Der erfindungsgemäße Regulatorvektor kodiert vorzugsweise für beide Regulatoren R₁ und R₂ der beiden Promotoren P₁ und P₂, welche sich auf dem erfindungsgemäßen Expressionsvektor befinden.

Als Regulatorvektor kommen beispielsweise Plasmide, Phagen, Cosmide, Phagen, Phasmide, Fosmide, Bacterial Artificial Chromsomes, Yeast Artificial Chromosomes, Viren und Retroviren (beispielsweise Vaccinia, Adenovirus, Adeno-associated Virus, Lentivirus, Herpes-Simplex-Virus, Epstein-Barr-Virus, Fowl Pox Virus, Pseudorabies, Baculovirus) und daraus abgeleitete Vektoren.

Der Regulatorvektor oder Teile davon können auch in das Genom integriert werden.

Jeder andere Vektor kann zur Herstellung des erfindungsgemäßen Regulatorvektors eingesetzt werden, solange er replizierbar und überlebensfähig im gewählten System (Wirt) ist.

Bevorzugt ist der Regulatorvektor ein Plasmid (im Rahmen der Erfindung als "Regulatorplasmid" bezeichnet).

Bevorzugt ist der erfindungsgemäße Expressionsvektor ebenfalls ein Plasmid, so dass das erfindungsgemäße Expressionssystem vorzugsweise zwei Plasmide umfasst: Expressionsplasmid und Regulatorplasmid.

In einer bevorzugten Ausführungsform umfasst das Regulatorplasmid mehr bp als der Expressionsvektor bzw. als das Expressionsplasmid.

In einer bevorzugten Ausführungsform handelt es sich bei dem erfindungsgemäßen

Regulatorplasmid um ein *low-copy* Plasmid (durchschnittlich < 100 Plasmide pro Zelle). In einer anderen bevorzugten Ausführungsform handelt es sich bei dem erfindungsgemäßen Regulatorplasmid um ein *high-copy* Plasmid (durchschnittlich ≥ 100 Plasmide pro Zelle).

Der Regulatorvektor enthält ebenfalls eine Selektionsmarkersequenz. Vorzugsweise unterscheidet sich die Selektionsmarkersequenz des Regulatorvektors von der Selektionsmarkersequenz des Expressionsvektors.

Der Regulatorvektor dient bevorzugt zur effektiven Kontrolle sowohl von P₁ als auch von P₂. Handelt es sich dabei um den *ara*-Promotor und den T7-Promotor, so stellt der Regulatorvektor bevorzugt ein um eine *araC*-Variation und einen Teil der *ara*-Regulatorregion erweiterten Vektor dar, welcher zusätzlich das Strukturgen für den Lacl-Repressor trägt.

AraC ist der Repressor/Aktivator des *ara*-Promotors, Lacl der Repressor des T7-Promotors.

Hierbei erfüllt der Lacl-Repressor zwei Funktionen. Zum einen bindet er an regulatorische Elemente zwischen T7-Promotor und Transkriptionsstart (Operatorsequenz *lacO*) und verhindert den Transkriptionsstart. Zum anderen steht in einer bevorzugten Ausführungsform die Expression der T7-RNA-Polymerase im Expressionswirt ebenfalls unter der Kontrolle einer *lacO*-Operatorsequenz. So lange der Lacl-Repressor an diese Operator-Sequenz bindet, wird die Expression der T7-RNA-Polymerase selbst unterdrückt und damit auch keine Sequenzen, welche unter Kontrolle des T7-Promotors stehen, transkribiert. IPTG (I₁) bindet an den lacl-Repressor, dieser wird dadurch inaktiviert und kann nicht mehr an die Operatorsequenzen *lacO* binden und damit die Transkription der T7-RNA-Polymerase selbst, als auch der stromabwärts des T7-Promotors liegenden Gene freigegeben.

Dies ermöglicht eine effektive Kontrolle der Expression durch IPTG- bzw. L-Arabinose-Induktion (Induktor I₁ bzw. Induktor I₂). Der erfindungsgemäße Expressionsvektor umfasst bevorzugt als Klonierungs- bzw. Expressionskomponente des 2-Komponentensystems auf der einen Seite der MCS die T7-Promotor/Operator-Region und auf der anderen Seite die komplette Ara-Promotor-Operator-Region (vgl. Figur 1)

In der Literatur wird durchgängig der *ara*-Regulator AraC auf dem gleichen Plasmid exprimiert wie das Zielgen. Dies ist bei erfindungsgemäßen Expressionsvektor vorzugsweise nicht so. Auf diese Weise wird ein maximal verkleinertes Plasmid erhalten, was Vorteile im Flaschenhals der Ligation/Transformation bringt, da die erzielbaren Transformationsraten und damit erzielbaren Bibliotheksgrößen umso größer sind, umso kleiner das verwendete Plasmid ist. Stattdessen kann *araC* in das T7-Regulatorplasmid kloniert werden, wo es analog zu *lacI* unabhängig vom Expressionsplasmid exprimiert wird. Gleichzeitig wird das *araC*-Gen bevorzugt verkürzt, um eine effizientere Induktorbindung zu gewährleisten. (Lee et al., (2007); Appl. Environ. Microbiol. 73, 5711-5715).

In einer besonderen Ausführungsform trägt der Regulatorvektor zusätzlich mindestens ein Gen für eine Transfer-RNA des Wirtsorganismus. Vorzugsweise sind diese Gene ausgewählt aus der Gruppe bestehend aus *argU, argw, ileX, gluT, leuW, proL, metT, thrT, tyrU, thrU* und *argX* von *E*. *coli,* welche die Codons AGG, AGA, AUA, CUA, CCC, GGA bzw. CGG erkennen. Durch das Vorhandensein dieser zusätzlichen Transfer-RNA-Gene können durch den Expressionsvektor auch solche Zielgene mit höherer Ausbeute exprimiert werden, die eine zu *E*. *coli* unterschiedliche Verwendung der Aminosäure-Codons in ihrer Sequenz (codon usage) aufweisen. Dies kann insbesondere für eukaryotische Gene (z.B. humane) oder Gene aus anderen Mikroorganismengruppen (z.B. Actinomyceten) vorkommen.

In einer weiteren besonderen Ausführungsform enthält der Regulatorvektor Gene für eines oder mehrere inhibitorische Proteine für eine oder mehrere RNA-Polymerasen. Bei dieser einen oder mehreren RNA-Polymerase handelt es sich um die verwendete(n) RNA-Polymerase, also um die RNA-Polymerase des Wirts und/oder einer in der Wirtszelle coexprimierten wirtsfremden RNA-Polymerase.

In einer weiteren besonderen Ausführungsform enthält das Expressionssystem, vorzugsweise der Regulatorvektor, das Gen *lysS,* welches das T7-Lysozym kodiert. Das T7- Lysozym kann an die T7-RNA-Polymerase binden und diese inaktivieren. Durch das Vorhandensein dieses Genes in der Wirtszelle wird eine basale Expression der T7-RNA-Polymerase unterdrückt und eine Expression findet erst statt, wenn die Expression der T7-RNA-Polymerase durch Zugabe eines externen Induktors (IPTG) erhöht wird und nicht mehr ausreichend T7-Lysozym zum binden vorhanden ist. Dadurch können auch sehr toxische Proteine unter Kontrolle des T7-Promotors exprimiert werden. Da wirtschaftliche relevante Enzyme häufig hydrolytische und damit toxische Aktivitäten darstellen (Proteasen, Lipasen ect.) ist dies von besonderem Vorteil.

Expressionsvektor und Regulatorplasmid sind erfindungsgemäß kompatibel und können vorzugsweise gleichzeitig im Wirt, z.B. in *E*. *coli,* repliziert werden. Zum Ablesen des T7-Promotors in *E*. *coli* ist die Expression von T7-Polymerase erforderlich, wie z.B. in *E*. *coli* BL21(DE3). Der *ara*-Promotor erfordert keine *E*. *coli*-fremde Polymerase.

Bevorzugt umfasst das erfindungsgemäße Regulatorplasmid insgesamt höchstens 7000 bp, bevorzugter höchstens 6500 bp, noch bevorzugter höchstens 6000 bp, am bevorzugtesten höchstens 5500 bp und insbesondere höchstens 5000 bp.

Besonders bevorzugt weist das erfindungsgemäße Regulatorplasmid wenigstens 70% Homologie zu <SEQ ID NO: 2> aufweist. Die Homologie wird hierbei bevorzugt mit Hilfe des Algorithmus nach Smith & Waterman (J Mol Biol., 1981, 147(1), 195-7) ermittelt, unter Verwendung der BLOSU M62-Matrix und Werten von 11.0 für die Eröffnung einer Lücke, bzw. 1.0 für die Erweiterung einer Lücke.

Ein weiterer Aspekt der Erfindung betrifft ein Verfahren zur Expression von DNA-Sequenzen unter Einsatz des vorstehend beschriebenen Expressionsvektors oder Expressionssystems umfassend die Schritte
(i) Transfizieren bzw. transformieren eines geeigneten Wirtsorganismus mit einem Regulatorvektor, der für einen Regulator R₁ von P₁ und /oder für einen Regulator R₂ von P₂ kodiert;
(ii) Klonieren einer DNA-Sequenz oder einer DNA-Sequenzmischung (Bibliothek) in den Expressionsvektor zwischen P₁ und P₂;
(iii) Transfizieren bzw. transformieren des in (i) erhaltenen Wirtsorganismus mit dem Regulatorvektor mit den in Schritt (ii) erhaltenen Konstrukten; und
(iv) Induzieren der Expression der durch die DNA-Sequenzen kodierten Proteine durch Zugabe eines Induktors I₁ für den Regulator R₁ oder eines Induktors I₂ für den Regulator R₂.

Ein weiterer Aspekt der Erfindung betrifft ein Verfahren zur Expression von DNA-Sequenzen unter Einsatz des vorstehend beschriebenen Expressionsvektors oder Expressionssystems umfassend die Schritte
a) Klonieren einer DNA-Sequenz oder einer DNA-Sequenzmischung (Bibliothek) in den Expressionsvektor zwischen P₁ und P₂;
b) Transfizieren bzw. Transformieren eins Wirtsorganismus, in welchem ein Regulatorvektor, der für einen Regulator R₁ von P₁ und/oder für einen Regulator R₂ von P₂ kodiert, oder Teile davon integriert sind, mit den in Schritt a) erhaltenen Konstrukten; und
c) Induzieren der Expression der durch die DNA-Sequenz kodierten Proteine durch Zugabe eines Induktors I₁ für den Regulator R₁ oder eines Induktors I₂ für den Regulator R₂.

Die DNA-Sequenz ist vorzugsweise Bestandteil einer (Meta)Genom-Bibliothek. Umfasst sind genomische DNA-Sequenzen, extrachromosomale DNA-Sequenzen und cDNA-Sequenzen.

In einer Ausführungsform erfolgt die Klonierung in den Expressionsvektor durch Subklonierung aus einem anderen Vektor.

Mit den Begriffen "Transfizieren" bzw. "Transformieren" im Sinne der Erfindung sind alle Methoden des Hineinbringens von Nukleinsäuren in den Wirt umfasst, z.B. auch Infizieren. Das Hineinbringen des Konstrukts kann, in Abhängigkeit vom verwendeten Wirt, auf verschiedene Art und Weise erfolgen. Das Hineinbringen des Konstrukts in einen prokaryotischen Wirt kann beispielsweise erfolgen mittels Transformation, z.B. Elektroporation, Transduktion oder Transfektion. Das Hineinbringen des Konstrukts in einen eukaryotischen Wirt kann, je nach Art des Konstrukts (Expressionsvektors) beispielsweise erfolgen über Calcium-Phosphat-DNA-Kopräzipitation, DEAE-Dextranvermittelte Transfektion,

Polybrene-vermittelte Transfektion, Elektroporation, Mikroinjektion, Liposomen-Fusion, Lipofektion, virale Infektion, retrovirale Infektion oder ballistische Verfahren.

Erfindungsgemäß kann auch der Regulatorvektor oder zumindest die Teile, die den Repressor kodieren, durch diese Verfahren in den Wirt hineingebracht werden.

In einer bevorzugten Ausführungsform des erfindungsgemäßen Verfahrens werden I₁ und I₂ zeitlich nacheinander zugegeben. Es wurde überraschend gefunden, dass auf diese Weise eine Inhibition des schwächeren Promotors vermieden werden kann.

In einer anderen bevorzugten Ausführungsform des erfindungsgemäßen Verfahrens werden I₁ und I₂ zu räumlich getrennten Teilkulturen der erhaltenen Organismen zugegeben und somit die beiden Promotoren einzeln induziert. Es wurde überraschend gefunden, dass auch auf diese Weise eine gegenseitige Inhibition der Promotoren vermieden werden kann.

Erfindungsgemäß erfolgt somit bevorzugt eine räumlich getrennte Induktion des Ablesens der gleichen Sequenz in unterschiedlichen Leserichtungen, nicht jedoch die zeitlich nacheinander oder gleichzeitige Induktion des Ablesens verschiedener Sequenzen.

Besonders bevorzugt ist I₁ Induktor für P₁, nicht jedoch für P₂, und/oder I₂ ist Induktor für P₂, nicht jedoch für P₁.

Ein weiterer Aspekt der Erfindung betrifft ein Verfahren zur Durchmusterung von DNA-Bibliotheken unter Einsatz des vorstehend beschriebenen Expressionsvektors oder Expressionssystems umfassend das vorstehend beschriebene Verfahren zur Expression von DNA-Sequenzen.

Bevorzugt erfolgt die Durchmusterung im Hinblick auf eine katalytische Aktivität der exprimierten Proteine. Bevorzugt handelt es sich um eine katalytische Aktivität einer der folgenden Enzymklassen: 1. Oxidoreduktasen, 2. Transferasen, 3. Hydrolasen, 4. Lyasen, 5. Isomerasen und 6. Ligasen. Bevorzugte Oxidoreduktasen sind ausgewählt aus der EC-Gruppe bestehend aus 1.1, 1.2, 1.3, 1.4, 1.5, 1.6, 1.7, 1.8, 1.9, 1.10, 1.11, 1.12, 1.13, 1.14, 1.15, 1.16, 1.17, 1.18, 1.19 und 1.97. Bevorzugte Transferasen sind ausgewählt aus der EC-Gruppe bestehend aus 2.1, 2.2, 2.3, 2.4, 2.5, 2.6, 2.7, 2.8 und 2.9. Bevorzugte Hydrolasen sind ausgewählt aus der EC-Gruppe bestehend aus 3.1, 3.2, 3.3, 3.4, 3.5, 3.6, 3.7, 3.8, 3.9, 3.10, 3.11 und 3.12. Bevorzugte Lyasen sind ausgewählt aus der EC-Gruppe bestehend aus 4.1, 4.2, 4.3, 4.4, 4.5, 4.6 und 4.99. Bevorzugte Isomerasen sind ausgewählt aus der EC-Gruppe bestehend aus 5.1, 5.2, 5.3, 5.4, 5.5 und 5.99. Bevorzugte Ligasen sind ausgewählt aus der EC-Gruppe bestehend aus 6.1, 6.2, 6.3, 6.4 und 6.5. Die von der International Union of Biochemistry and Molecular Biology (IUBMB) eingeführte EC-Nomenklatur ist dem Fachmann bekannt. Informationen hierzu finden sich auf Website des IUBMB.

Geeignete Assay zum Auffinden einer gegebenen katalytischen Aktivität sind dem Fachmann bekannt. Vorzugsweise basieren sie auf UV/VIS-Spektroskopie, Fluoreszenz, Lumineszenz oder Radioaktivität. In diesem Zusammenhang kann beispielsweise vollumfänglich verwiesen werden auf J.L. Reymond, Enzyme Assays: High-throughput Screening, Genetic Selection and Fingerprinting, Wiley VCH, 2006.

Alternativ ist jedoch auch eine Durchmusterung auf Grundlage von Bindungsaffinitäten möglich. Hierbei kann es sich beispielsweise um die Bindungsaffinität zu einem Antikörper oder zu einem sonstigen Bindungspartner (beispielsweise ein Protein oder eine Nukleinsäure oder ein Kohlenhydrat) handeln.

Auch ist eine Durchmusterung auf Grundlage von jeweils geeigneten, dem Fachmann bekannten funktionellen Assays möglich.

In einer Ausführungsform erfolgt die Identifikation der selektierten Sequenz durch Sequenzierung der klonierten Sequenz.

In einer besonderen Ausführungsform des Verfahrens wird die Wirtszelle vermehrt und das exprimierte Protein kann weiteren Schritten wie einer Aufreinigung und/oder einer biochemischen und/oder funktionellen Charakterisierung zugeführt werden.

In einer besonderen Ausführungsform erfolgen diese Schritte unter Verwendung der mit dem exprimierten Protein verbundenen Tags. Als Tags können beispielsweise ein His-tag, ein Poly-His-tag, ein MAT-tag, ein Streptavidin-tag, ein Streptavidine-binding-tag, ein GST-tag, ein Antikörper-Bindungs-tag, ein Myc-tag, ein Swa11-Epitop oder ein FLAG-tag oder fluoreszierende Tags wie ein GFP-tag, ein BFP-tag oder ein RFP-tag verwendet werden.

Das bevorzugte Einsatzgebiet des erfindungsgemäßen Expressionsvektors ist als Klonierungs- und Expressionsvektor für das Enzymaktivitäts-Screening von Genom- und Metagenom-Bibliotheken. Gerade bei (Meta)genombibliotheken ist eine hohe Komplexität (> 10⁶ Klone) notwendig, so dass bereits bei deren Erstellung eine große Klonierungs- und Transformationseffizienz entscheidend ist. Weiterhin muss der ideale Screeningvektor ermöglichen, dass die hohen Klonzahlen auch effizient durchmustert werden können. Beim *Cluster Screening* wie auch bei anderen Durchmusterungsansätzen ist eine starke, regulierbare Expression der Zielproteine essentiell. Der erfindungsgemäße Expressionsvektor wurde speziell für diese Anforderungen - hohe Klonierungseffizienz verbunden mit effizienter, regulierbarer Expression - entwickelt.

Im Gegensatz zu den aus dem Stand der Technik bekannten Systemen besitzt der erfindungsgemäße Expressionsvektor zwei starke, plasmidständige Promotoren, die außerdem noch regulierbar sind, was Vorteile beim Screening nach leicht toxischen Proteinen bietet. Im Falle leicht toxischer Proteine verträgt nämlich der Wirtsorganismus, z.B. *E*. *coli,* die Gegenwart dieser Proteine nur für relativ kurze Zeit. In solchen Fällen erlauben es regulierbare Promotoren, das Gen, welches für diese leicht toxischen Proteine codiert, zunächst "auszuschalten", bis sich der Wirtsorganismus hinreichend stark vermehrt hat. Danach erlauben es die regulierbaren Promotoren, das Gen "anzuschalten" und so die Produktion der leicht toxischen Proteine für einige Zeit zu induzieren, ehe die exprimierten Proteine ihre toxische Wirkung entfalten. Neben der möglichen Toxizität eines Zielproteins stellt i. A. jede zusätzliche Expression eines rekombinanten Proteins eine Belastung für den Wirtsorganismus dar (Verbrauch von Ressourcen). Daher ist es i.d.R. immer vorteilhaft, die Expression der rekombinanten Proteine erst nach dem Erreichen einer ausreichend starken Vermehrung anzuschalten.

Mit den zwei konvergenten Promotoren im erfindungsgemäßen Expressionsvektor ist es möglich, beide potentiellen Orientierungen zu erfassen und den nutzbaren Informationsgehalt der klonierten DNA so zu verdoppeln. Die ORFs können orientierungsunabhängig exprimiert und damit ihre Genprodukte aktivitätsbasierend gescreent werden.

Neben der großen Promotorstärke ist auch die getrennte Induktion der beiden Promotoren vorteilhaft, da so mögliche antisense RNA-Effekte ausgeschlossen werden können.

Die getrennt induzierbaren Promotoren des erfindungsgemäßen Expressionsvektors bieten Vorteile. Eine Verminderung der Promotorstärke, bzw. der Expressionseffizienz der abgelesenen ORFs kann so vermieden werden.

Transkriptionelle Interferenzen durch aufeinander zulaufende Promotoren wurden bei Eukaryoten bereits beobachtet. So beschreiben *Callen et al.* eine Unterdrückung des schwächeren Promotors um Faktor 5,6 bei dicht aneinander liegende face-to-face Promotoren unterschiedlicher Stärke (Callen et al. (2004), Molecular Cell, 14, 647-56 B). *Eszterhas et al.* zeigen, dass bei einem konvergenten Promotorarrangement die Aktivität zweier Reportergene beinahe auf das Hintergrundlevel reduziert wird (Eszterhas et al. (2002), Molecular and Cellular Biology 22, 469-79). Dies wir u. a. auf eine Störung der Bindeeigenschaften in der Promotorregion zurückgeführt. Eine Übertragung dieser Ergebnisse auf Prokaryoten ist unter Berücksichtigung ihrer von Eukaryoten unterschiedlichen Transkriptionsinitiation mit Einschränkungen möglich.

Das erfindungsgemäße Expressionssystem verbindet die geringe Größe eines klassischen Klonierungsvektors mit den Expressionsmöglichkeiten von regulierbaren Expressionsvektoren. Durch die Verwendung der beiden konvergenten Promotoren wird die Bibliotheksgröße, welche durchmustert werden muss, um eine bestimmte Menge DNA statistisch abzudecken, halbiert. Die separate Induktion der Promotoren verhindert eine mögliche transkriptionelle Interferenz durch antisense RNA, die bei gleichzeitiger Induktion zwangsläufig gebildet wird bzw. eine durch eine höhere Transkriptionsrate des stärkeren Promotors herabgesetzte Transkriptionsaktivität vom schwächeren Promotor.

Eine große, gut regulierbare Promotorstärke bringt den entscheidenden Vorteil im *Cluster Screening* Verfahren, da die starken Signale vor dem Hintergrund besser detektiert werden und dadurch größerer Komplexitäten als bisher durchmustert werden können.

Damit ist ist das erfindungsgemäße Expressionssystem maßgeschneidert für jede Art des Aktivitätsscreenings von Banken mit randomisiert fragmentierter (meta)genomischer DNA, insbesondere aber für das *Cluster Screening.*

Hierbei handelt es sich um ein Verfahren zur iterativen Dekonvolution von Varianten-Bibliotheken, welches erhebliche Vorteile gegenüber herkömmlichen Dekonvolutionsverfahren aufweist.

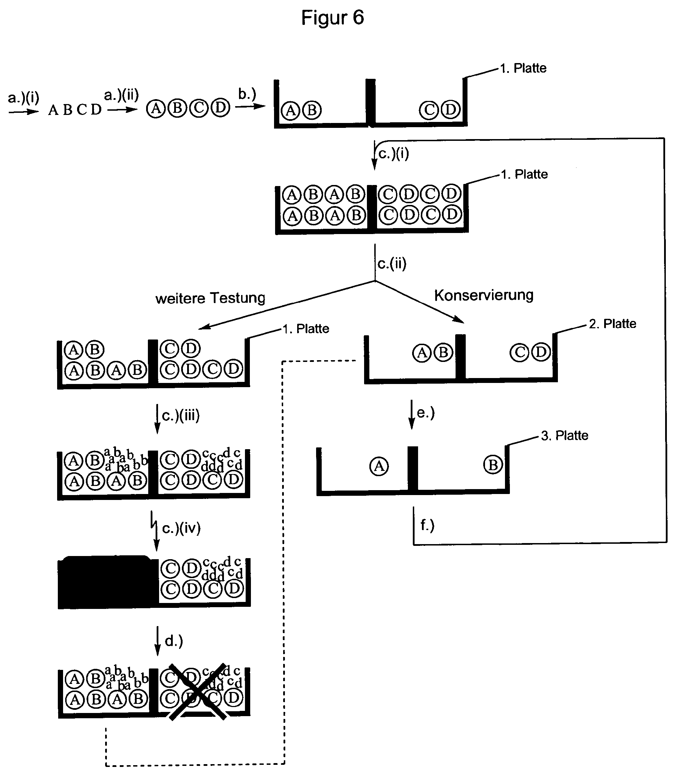

In einer bevorzugten Ausführungsform eines solchen Verfahrens, schematisch illustriert in Figur 6, wird eine Bibliothek, vorzugsweise eine (Meta)Genom-Bibliothek, hergestellt (Figur 6, Schritt a.)(i)). Die Bibliothek enthält hier die einzelnen Varianten "A", "B", "C" und "D". Erfindungsgemäß wird diese Bibliothek in einen Wirt überführt (Figur 6, Schritt a.)(ii)).

In Schritt b.) werden die Klone einer Teilbibliothek in ein erstes Kompartiment (Varianten "A" und "B" in Figur 6) und die Klone einer anderen Teilbibliothek in ein zweites Kompartiment (Varianten "C" und "D" in Figur 6) aufgeteilt.

Bei dieser Aufteilung ist nicht bekannt, welche Varianten in welches Kompartiment gegeben werden. Die Kompartimente können beispielsweise zwei benachbarte Vertiefungen auf einer ersten Mikrotiterplatte ("1. Platte") sein.

Nun erfolgt in Schritt c.)(i) eine Vervielfältigung der Klone der einzelnen Teilbibliotheken, vorzugsweise durch Wachstum der Organismen innerhalb der Kompartimente auf der 1. Platte.

In einer bevorzugten Ausführungsform wird im Anschluss daran in Schritt c.)(ii) ein Aliquot der vervielfältigten Organismen vorzugsweise unter Beibehaltung der Kompartiment-Zuordnung konserviert. Zur Beibehaltung der Kompartiment-Zuordnung kann beispielsweise eine zweite Mikrotiterplatte ("2. Platte") verwendet werden, wobei vorzugsweise das Aliquot der vervielfältigten Organismen, welches dem ersten Kompartiment auf der 1. Platte entnommen wird, in das entsprechende erste Kompartiment auf der 2. Platte überführt wird.

Mit dem nicht-konservierten Teil der vervielfältigten Organismen werden in Schritt c.)(iii) Biomoleküle produziert, wobei Klone, welche die Variante "A" enthalten, Biomoleküle "a" produzieren; Klone, welche die Variante "B" enthalten, Biomoleküle "b" produzieren; usw. Typischerweise handelt es sich bei den Biomolekülen um Proteine, welche von den Organismen exprimiert werden. Die Wirtsorganismen werden aufgeschlossen. Dem Fachmann sind hierzu verschiedenen Methoden bekannt, wie beispielsweise die Zelllyse durch geeignete Chemikalien oder die Zelllyse durch osmotischen Schock oder durch Anwendung von Scherkräften wie bei dem "French-Press"-Verfahren. Die Folge ist eine Entkopplung von Phänotyp und Genotyp.

In Schritt c.)(iv) wird nun jeweils die Gesamtheit der in dem ersten Kompartiment enthaltenen Biomoleküle "a" und "b" und die Gesamtheit der in dem zweiten Kompartiment enthaltenen Biomoleküle "c" und "d" getestet. Dieses erfolgt vorzugsweise durch einen Screen auf eine bestimmte biokatalytische Aktivität (Phänotyp). Im gewählten Beispiel zeigt nur die Gesamtheit der im ersten Kompartiment enthaltenen Biomoleküle "a" und "b" die gewünschte biokatalytische Aktivität, was durch eine graue Unterlegung des ersten Kompartiments symbolisiert wird. Dabei sind aus dem beobachteten Phänotyp keine direkten Rückschlüsse auf den Genotyp möglich, da nach außen nicht ersichtlich ist, welches der Biomoleküle für die positive Testung verantwortlich ist, "a" oder "b", und auch nicht bekannt ist, aus welchen Varianten sich die Gesamtheit der Teilbibliothek zusammensetzt (vgl. Erläuterung Schritt b.) oben).

Das erste Kompartiment enthält somit Biomoleküle, welche die gewünschte biokatalytische Aktivität erfüllen, und wird in Schritt d.) ausgewählt.

Das weitere Verfahren geht nun vorzugsweise nicht von der ausgewählten Teilbibliothek im ersten Kompartiment als solche aus, sondern von der konservierten Teilbibliothek im entsprechenden ersten Kompartiment auf der 2. Platte (durch eine gestrichelte Linie angedeutet). Es ist auch möglich, die Konservierung der Teilbibliotheken direkt in der 1. Platte durchzuführen. In Schritt e.) wird die konservierte Teilbibliothek, welche die Klone der Varianten "A" und "B" umfasst, verdünnt und aufgeteilt. Dabei werden die Klone der Varianten "A" und "B" in jeweils unterschiedliche Kompartimente überführt. Die Kompartimente können beispielsweise zwei Vertiefungen auf einer dritten Mikrotiterplatte ("3. Platte") sein.

Schließlich werden in Schritt f.) die Schritte c.) bis e.) sooft wiederholt, bis in jedem Kompartiment nur noch maximal eine Variante der für das Biomolekül codierenden Gensequenz enthalten ist. Unter diesen Voraussetzungen sind dann aus dem beobachteten Phänotyp direkte Rückschlüsse auf den Genotyp möglich, da alle in dem Kompartiment enthaltenen Biomoleküle auf einen einzelnen, separierten Klon zurückgehen.

In einer besonderen Ausführungsform des erfindungsgemäßen Verfahrens zur Durchmusterung von DNA-Bibliotheken umfasst die DNA-Bibliothek 10³ bis 10²⁵ verschiedene Sequenzen. Die DNA-Bibliothek kann beispielsweise 10³ bis 10⁵, 10⁵ bis 10¹⁰, 10¹⁰ bis 10¹⁵, 10¹⁵ bis 10²⁰ oder auch 10²⁰ bis 10²⁵ verschiedene Sequenzen umfassen.

Erfindungsgemäß können die Schritte c.) bis e.) wiederholt werden, wobei der Fachmann in der Lage ist, unter Berücksichtigung der Größe der Bibliothek, eine für die jeweiligen Umstände angemessene Anzahl von Wiederholungen zu bestimmen.

Erfindungsgemäß können die Schritte c.) bis e.) beispielsweise mindestens 1x, vorzugsweise mindestens 2x, bevorzugter mindestens 3x, noch bevorzugter mindestens 5x, noch bevorzugter mindestens 10x wiederholt werden bis einzelne Sequenzen individualisiert werden.

In einer bevorzugten Ausführungsform enthält jedes Kompartiment nach der ersten Aufteilung der Bibliothek auf Kompartimente der 1. Platte durchschnittlich mindestens 10, bevorzugter mindestens 20, noch bevorzugter mindestens 40, am bevorzugtesten mindestens 100 und insbesondere mindestens 1000 verschiedene Varianten. In einer Ausführungsform umfassen somit die Teilbibliotheken in der ersten Runde vorzugsweise ≥ 10, bevorzugter ≥ 10², bevorzugter ≥ 10³ Sequenzen.

Die nachfolgenden Beispiele dienen zur Erläuterung der der Erfindung, sind jedoch nicht einschränkend auszulegen.

Bei den nachfolgenden Beispielen wurde pF2F4 eingesetzt, ein Expressionsvektor für E. *coli,* bei dem zwei starke Promotoren die *multiple cloning site* flankieren (vgl. Figur 1). Die Promotoren sind konvergent, d.h. ihre Leserichtung ist aufeinander zulaufend (*face-to-face*)*.* Bei den unabhängig voneinander induzierbaren Promotoren handelt es sich um einen T7-Promotor und einen Arabinose-Promotor. In diesen Vektor klonierte DNA kann also von beiden Seiten transkribiert werden, was die Zahl der zu durchmusternden Klone halbiert. Die starke vektorgestützte Transkription ist unabhängig von insertcodierten Promotoren und erhöht so die Hitquote.

### Beispiel 1:

Mit Hilfe eines Reportergens wurde die Promotorstärke des *ara-* bzw. T7-Promotors in pF2F4 in verschiedenen Situationen untersucht. Die Daten zeigen, dass pF2F4 im Zusammenspiel mit dem Regulatorplasmid pLacl+ (vgl. Figur 2) optimal als Expressionsplasmid eingesetzt werden kann. Als Reportergen wurde eine Alkohol-Dehydrogenase (ADH) verwendet, die in beiden möglichen Orientierungen inseriert wurde. Das Gen stand entsprechend unter Kontrolle des *Ara*-Promotors oder des T7-Promotors. Erst die Kombination von Regulatorplasmid codiertem Lacl und AraC mit dem pF2F4-Plasmid führt zu einer größtmöglichen Expression vom *Ara*-Promotor als auch vom T7-Promotor aus (Figur 3).

Die ara-Promotor-Aktivität wird im BL21(DE3) Stamm bei gleichzeitiger T7-Induktion auf ca. 10 % der Ausgangsaktivität gesenkt wird (Figur 4A). Es kann ausgeschlossen werden, dass dieser Effekt auf einer kompetitiven Hemmung des Regulators AraC durch IPTG beruht, da die Hemmung nur in *E*. *coli* BL21(DE3) beobachtet wird. In einem *E*. *coli* Stamm ohne chromosomale T7-Polymerase (DH10B) ist kein signifikanter Rückgang der *ara*-Promotor Aktivität zu beobachten (Figur 4B). Hier ist die T7-Aktivität weitestgehend ausgeschaltet. Die dennoch auftretende minimale Aktivität ist die basale Aktivität des T7-Promotors, der auch in *E. coli* ohne chromosomale T7-Polymerase von der dem Wirtsorganismus eigenen Polymerase in geringem Umfang erkannt wird (Figur 4).

### Beispiel 2: - Anwendungsbeispiel pF2F4: Screening nach Esterase/Lipase Aktivität in einer Metagenombank

Eine in pF2F4 erstellte Metagenombibliothek wurde nach Esterase/Lipase-Aktivität durchmustert, wobei das *Cluster Screening* Verfahren (Greiner-Stoeffele, T., Struhalla, M., 2005, WO2004002386) zur Anwendung kam. Dabei wurde die Hitrate mit der einer in den herkömmlichen pUC-Vektor klonierten Metagenombank verglichen. Bei der Zielaktivität handelte es sich um eine mit einem etablierten Enzymtest gut nachzuweisende Aktivität, deren Vorkommen in Metagenombanken in der Literatur hinreichend beschrieben ist.

### 1. Erstellung der Metagenombank

Für die verwendete Metagenombanken wurde metagenomischer DNA (mgDNA) aus dem Inhalt eines Schafspansens mit der direct-lysis Methode isoliert (Zhou,J.; Bruns,M.A.; Tiedje,J.M. (1996): DNA recovery from soils of diverse composition. Appl Environ.Microbiol; 62(2): 316-22). Für die Erstellung der Metagenombank in pF2F4 wurde die mgDNA anschließend mit dem Restriktionsenzym Alul partiell verdaut und nach Standardmethoden in den mit HincII und EcoRV *blunt-end* geschnittenen und dephosphorylierten Vektor pF2F4 ligiert (Sambrook, J., Fritsch, E.F., Maniatis, T., (1989). Molecular cloning: A labatory manual. Cold Spring labatory Press 2nd Ed. Cold Spring Harbor, USA).

Zur Erstellung der Metagenombank in pUCWhite, einem pUC18-Derivat, wurde die mgDNA mit Bsp143I verdaut und ebenfalls mit Standardmethoden in den BamHI geschnittenen und dephosphorylierten Vektor pUCWhite ligiert.

Zur Vervielfältigung der Bibliotheken wurden elektrokompetente *E*. *coli* DH10B Zellen mittels Elektroporation mit den Bibliotheken transformiert. Die pF2F4-Bibliothek hatte eine durchschnittliche Insertgröße von 3,7 kb mit Inserts von 2,4-4,6 kb und eine Größe von 2,9 x 10⁶ individuellen Klonen. Die pUC-Bibliothek hatte eine durchschnittliche Insertgröße von 3,5 kb mit Inserts von 1,9-5,9 kb und eine Größe von 3,9 x 10⁶ individuellen Klonen. Nach Überprüfung der Qualität wurden die Bibliotheken mittels Präparation im Midi-Maßstab (Qiagen, Hilden) aus *E*. *coli* DH10B isoliert und elektrokompetente Zellen des Expressionsstamms *E*. *coli* BL21 (DE3) mit 720 ng (pF2F4-Pansen) bzw. 200 ng (pUC-Pansen) der Bibliothek transformiert. Der mit der pF2F4 Bibliothek transformierte Expressionsstamm enthielt zusätzlich das Regulatorplasmid pLacl+.

### 2. Zellanzucht

Bei der Durchmusterung der Metagenombanken wurde das Clusterscreening-Verfahren angewendet (Greiner-Stoeffele, T., Struhalla, M., 2005, WO2004002386). Bei diesem High-Throughput-Verfahren werden in den initialen Durchmusterungen Mischkulturen (Cluster) von bis zu 1000 individuellen Klonen (hier 300) angelegt. Die Cluster, auf die in diesem ersten Durchmusterungsschritt gefundene Hits zurückgehen, werden verdünnt und neu durchmustert, bis man auf Einzelklonebene angekommen ist. Die erhaltenen Einzelklone werden dann enzymatisch und molekularbiologisch charakterisiert. In diesem Anwendungsbeispiel wird nur die initiale Durchmusterung durchgeführt. Alle Anzuchten wurden unter für das jeweilige Expressionssystem optimierten Bedingungen durchgeführt. Da der pF2F4-Vektor zwei konvergente Vektoren besitzt, und diese getrennt induziert werden sollten, wurden von der pF2F4-Bibliothek aus einer Vorkultur zwei Hauptkulturen mit Standardmedien angeimpft.

### 2. a Vorkultur

Die Kultivierung der Bibliotheken im Expressionsstamm erfolgte im 96-well Format in Deep-well-Platten. Dabei wurde zunächst eine Vorkultur angezogen. Jedes well wurde mit ∼300 individuellen Klonen einer Metagenombank beimpft, wobei jedoch well A1 als Kontrolle unbeimpft blieb. Gleichzeitig wurden Aliquots des beimpften Kulturmediums ausplattiert um die Klonzahl zu verifizieren. Dabei wurden für die pF2F4-Pansen Bank 278 individuelle Klone/well nachgewiesen und für die pUC-Pansenbank 300 individuelle Klone/well. Die Vorkultur erfolgte in 400 µl Medium. Bei der Vorkultur der pUC-Bibliothek wurden dem Medium 1 % Glucose und 100µg/ml Ampicillin zugesetzt, Bei der Vorkultur der pF2F4-Bibliothek wurden dem Medium 0,5 % Glucose sowie 50 µg/ml Kanamycin und 37 µg/ml Chloramphenicol zugesetzt. Die Anzucht erfolgte ü/N bei 37°C und 1000 Upm in einem Rundschüttler.

### 2.b Hauptkultur

Für die Hauptkultur der pF2F4-Bibliothek wurden zwei Deepwell-Platten parallel beimpft, da die konvergenten Promotoren pAra und pT7 getrennt induziert werden sollten. Die Hauptkulturen der pUC-Bibliothek und der später mit IPTG zu induzierende Teil der pF2F4-Bibliothek wurden in 1,2 ml Medium mit 0,5 % Glucose und den entsprechenden Antibiotika (Ampicillin für die pUC-Bibliothek und Kanamycin und Chloramphenicol für die pF2F4-Bibliothek) angezogen. Der mit Arabinose zu induzierende Teil der pF2F4-Bibliothek wurde im gleichen Medium ohne Glucose angezogen. Die Inokulation der Hauptkulturen erfolgte mit jeweils 30 µl Vorkultur, wobei well A1 als Kontrolle unbeimpft blieb. Nach einer Inkubation bei 30 °C und 1000 Upm wurden die Kulturen bei Erreichen einer OD von 0,7 induziert. Dazu wurden der pUC-Bibliothek 1 mM IPTG und den beiden pF2F4-Platten 0,5 mM IPTG bzw. 0,2 % L-Arabinose zugegeben. Die weitere Kultivierung erfolgte ü/N bei 30 °C und 1000 Upm.

### 3. Zellernte und -aufschluss

Die über Nacht gewachsenen Expressionskulturen wurden bei 4000 x g abzentrifugiert. Der Kulturüberstand wurde abgenommen, um diesen zusätzlich zum Zellextrakt im Enzymtest einzusetzen. Die Zellpellets wurden zur Gewinnung des Zellextraktes in CellLytic-Puffer aufgeschlossen. Dazu wurden sie in je 200 µl CellLytic-Puffer resuspendiert und 30 min bei 37 °C inkubiert. Anschließend wurden die Zelltrümmer 15 min lang bei 4°C mit 4000 x g abzentrifugiert.

CellLytic-Puffer:
1 ml CellLytic B Cell Lysis Reagent (Sigma-Aldrich, Steinheim)
1 mg Lysozym (Applichem, Darmstadt)
1 µl Benzonase (Sigma-Aldrich)
ad 10 ml 50 mM K-Phosphat-Puffer pH 8

### 4. Enzymaktivitätstest

Die Aktivitätstests erfolgten mit pNP-Caprylat, einem künstlichen Substrat, bei dem eine aus 8 C-Atomen bestehende Fettsäure über eine Esterbindung mit para-Nitrophenol derivatisiert ist. Bei Abbau wird p-Nitrophenolat frei, welches bei 405 nm nachweisbar ist. Es wurden jeweils 5 µl Zellextrakt bzw. 5 µl Kulturüberstand mit 95 µl Assaypuffer in Flachboden 96-well-Platten vermischt und bis zu 12 h bei Raumtemperatur inkubiert. Bei zu hohen Hintergrundwerten wurden die Zellextrakte in KPBT-Puffer 1:10 verdünnt. Anschließend wurde die Absorption bei 405 nm in einem Mikroplatten-Reader (Infinite 200, Tecan, Crailsheim) bestimmt.

Zusammensetzung Assaypuffer:
200 µl pNP-Caprylat (Sigma-Aldrich)
ad 20 ml KPBT-Puffer
   KPBT-Puffer:
      23,5 ml 1 M K2HPO4
      1,5 ml 1 M KH2PO4
      2,5 ml 20 % Triton X-100
      ad 500,0 ml AquaMP
      pH 8,0

### 5. Auswertung

Als Hit wurden wells gewertet, bei denen der Faktor Z > 4 war, wobei Z wie folgt definiert ist: Z = (Absorptionszunahme des Wells - Durchschnitt der Absorptionszunahme der gesamten 96well-Platte) / Standardabweichung des Durchschnitts der Absorptionszunahme der gesamten 96well-Platte.

### Ergebnisse

Von der pF2F4-Pansen Bibliothek wurden ∼ 26400 Klone mit einer Gesamtinsertgröße von 97,7 Mb auf Esterase/Lipaseaktivität durchmustert. Dabei wurden sowohl die Kulturüberstände als auch die Zellextrakte beider Induktionschargen betrachtet. Es ergaben sich 10 nicht redundante Hits, was eine Hitrate von 1 Hit/9,8 Mb entspricht. Hits, die sich in mehreren Messungen zeigten, fließen nur einfach in die Gesamtbilanz ein.

Von der pUC-Pansen Bibliothek wurden ∼28500 Klone mit einer Gesamtinsertgröße von 99,8 Mb auf Esterase/Lipaseaktivität durchmustert. Dabei wurden sowohl die Kulturüberstände als auch die Zellextrakte betrachtet. Es ergab sich 1 Hit, was einer Hitrate von 1 Hit/99,8 Mb entspricht. Damit ergibt sich für die Metagenombibliothek in pF2F4 eine ∼10fach höhere Hitrate als für die pUC-Bibliothek. In Tabelle 1 sind die Hits zusammengestellt, Figur 5 zeigt die Hitverteilung im Zellaufschluss der mit IPTG induzierten pF2F4-Bibliothek.

**Tabelle 1: Esterase/Lipase Hits in den Bibliotheken nach bis zu 24 h Inkubation mit pNP-Caprylat**

| | pF2F4-Pansen (97 Mb durchmustert) | pUC-Pansen (95 Mb durchmustert) |
|---|---|---|
| Kulturüberstände, IPTG-induziert | 1 | 0 |
| Zellaufschluss, IPTG-induziert | 6 | 1 |
| Kulturüberstände, Arabinose-induziert | 0 | - |
| Zellaufschluss, Arabinose-induziert | 5 | - |
| Summe | 12 | 1 |
| Summe abzüglich mehrfach auftretender Hits | 10 | 1 |

### Hitratenvergleich

Um zu zeigen, dass das 2-Promotorsystem in pF2F4 einem einfachen *lac*-Promotor überlegen ist, wurde ein Hitratenvergleich durchgeführt. Dazu wurde ein Testscreening auf Lipase/Esterase-Aktivität mit pNP-Caprylat als Substrat im *Cluster Screening* mit ∼300 Klonen/well durchgeführt. Bei den verwendeten Bibliotheken handelt es sich um fragmentierte metagenomische DNA, die aus der Schafspansenflora gewonnen wurde und die sowohl in pF2F4 als auch in pUCwhite, ein pUC18 Derivat, kloniert wurde. Die durchschnittlichen Insertlängen betrugen 3,5 kb (pUC-Pansen) bzw. 3,7 kb (pF2F4-Pansen). Im vergleichenden Screening wurden somit 101 Mb bzw. 99 Mb klonierte DNA abgedeckt. In diesem Test-Screening zeigte sich, dass durch die Kombination von starken Promotoren und Promotorkonvergenz bei gleicher Insert-DNA und Screeningmethode eine gegenüber einem einseitigen *lac*-Promotor System (pUC-Vektor) um Faktor 9,5 höhere Hitrate (1/9,7 Mbp zu 1/92 Mbp) erzielt werden kann. Da durch die konvergente Anordnung der Promotoren nur eine doppelt so hohe Hitrate zu erwarten wäre, muss die restliche Steigerung der Hitrate auf die Promotorstärke zurückzuführen sein.

## Patentansprüche

1. Ein Expressionsvektor umfassend zwei unabhängig voneinander induzierbare, aufeinander zulaufende Promotoren P₁ und P₂;
wobei zwischen P₁ und P₂ jeweils stromabwärts eine Insertionssequenz angeordnet ist, so dass die Expression einer in die Insertionssequenz klonierten DNA-Sequenz unter die Kontrolle von P₁ und P₂ gestellt wird;
wobei die Insertionssequenz ein Polylinker und/oder eine Sequenz ist, welche eine Integration von DNA-Sequenzen mittels Rekombination ermöglicht;
wobei der Expressionsvektor ohne die Insertionssequenz insgesamt höchstens 3000 bp umfasst; und
wobei der Expressionsvektor nicht für einen Regulator R₁ von P₁ und nicht für einen Regulator R₂ von P₂ kodiert.

2. Der Expressionsvektor nach Anspruch 1, **dadurch gekennzeichnet, dass** P₁ ein *T7* Promotor und/oder P₂ ein *Ara* Promotor ist

3. Der Expressionsvektor nach Anspruch 1 oder 2, **dadurch gekennzeichnet, dass** er für jeweils mindestens einen Terminator T₁ bzw. T₂ in entsprechender Leserichtung der Promotoren P₁ bzw. P₂ kodiert.

4. Der Expressionsvektor nach Anspruch 3, **dadurch gekennzeichnet, dass** T₁ ein *T7* Terminator ist und/oder T₂ ein Terminator für die Wirts-RNA-Polymerase ist, oder umgekehrt.

5. Der Expressionsvektor nach Anspruch 3 oder 4, **dadurch gekennzeichnet, dass** zwischen P₁ und seinem Terminator T₁ in Leserichtung von P₁ ein zusätzliches Gen liegt oder dass zwischen P₂ und seinem Terminator T₂ in Leserichtung von P₂ ein zusätzliches Gen liegt.

6. Ein Expressionssystem umfassend einen Expressionsvektor und einen Regulatorvektor, wobei der Expressionsvektor definiert ist wie in einem der Ansprüche 1 bis 5, und wobei der Regulatorvektor für einen Regulator R₁ von P₁ und/oder für einen Regulator R₂ von P₂ kodiert.

7. Das Expressionssystem nach Anspruch 6, **dadurch gekennzeichnet, dass** R₁ *LacI* und/oder R₂ *AraC* ist.

8. Das Expressionssystem nach Anspruch 6 oder 7, **dadurch gekennzeichnet, dass** der Regulatorvektor zusätzlich mindestens ein Gen für Transfer-RNAs des Wirtsorganismus enthält.

9. Das Expressionssystem nach Anspruch 8, **dadurch gekennzeichnet, dass** das Transfer-RNA-Gen ausgewählt ist aus der Gruppe bestehend aus argU, argW, ileX, gluT, leuW, proL, metT, thrT, tyrU, thrU und argX von *E*. *coli,* welche die Codons AGG, AGA, AUA, CUA, CCC, GGA bzw. CGG erkennen.

10. Das Expressionssystem nach Anspruch 9, **dadurch gekennzeichnet, dass** es das Gen LysS für das T7 Lysozym enthält.

11. Ein Verfahren zur Expression von DNA-Sequenzen unter Einsatz des Expressionsvektors nach einem der Ansprüche 1 bis 5 oder des Expressionssystems nach einem der Ansprüche 6 bis 10 umfassend die Schritte
(i) Transfizieren bzw. Transformieren eines geeigneten Wirtorganismus mit einem Regulatorvektor, der für einen Regulator R₁ von P₁ und/oder für einen Regulator R₂ von P₂ kodiert;
(ii) Klonieren einer DNA-Sequenz oder einer DNA-Sequenzmischung (Bibliothek) in den Expressionsvektor zwischen P₁ und P₂;
(iii) Transfizieren bzw. Transformieren des in (i) erhaltenen Wirtorganismus mit dem Regulatorvektor mit den in Schritt (ii) erhaltenen Konstrukten; und
(iv) Induzieren der Expression der durch die DNA-Sequenzen kodierten Proteine durch Zugabe eines Induktors I₁ für den Regulator R₁ oder eines Induktors I₂ für den Regulator R₂.

12. Ein Verfahren zur Expression von DNA-Sequenzen unter Einsatz des Expressionsvektors nach einem der Ansprüche 1 bis 5 oder des Expressionssystems nach einem der Ansprüche 6 bis 10 umfassend die Schritte
a) Klonieren einer DNA-Sequenz oder einer DNA-Sequenzmischung (Bibliothek) in den Expressionsvektor zwischen P₁ und P₂;
b) Transfizieren bzw. Transformieren eines Wirtsorganismus, in welchem ein Regulatorvektor oder Teile davon in das Genom integriert sind, die für einen Regulator R₁ von P₁ und/oder für einen Regulator R₂ von P₂ kodieren, mit den in Schritt a) erhaltenen Konstrukten; und
c) Induzieren der Expression der durch die DNA-Sequenzen kodierten Proteine durch Zugabe eines Induktors I₁ für den Regulator R₁ oder eines Induktors I₂ für den Regulator R₂.

13. Das Verfahren nach einem der Ansprüche 11 oder 12, **dadurch gekennzeichnet, dass** I₁ und I₂ zu räumlich getrennten Teilkulturen der erhaltenen Wirtsorganismen mit Regulator- und Expressionsvektor zugegeben werden.

14. Ein Verfahren zur Durchmusterung von DNA-Bibliotheken unter Einsatz des Expressionsvektors nach einem der Ansprüche 1 bis 5 oder des Expressionssystems nach einem der Ansprüche 6 bis 10 umfassend das Verfahren nach einem der Ansprüche 11 bis 13.

15. Das Verfahren nach Anspruch 14, **dadurch gekennzeichnet, dass** die Durchmusterung im Hinblick auf eine katalytische Aktivität der exprimierten Proteine erfolgt.

## Claims

1. An expression vector containing two mutually independently inducible, convergent promoters P₁ and P₂;
wherein between P₁ and P₂ an insertion sequence is positioned downstream of each, so that the expression of a DNA sequence cloned into the insertion sequence is placed under the control of P₁ and P₂;
wherein the insertion sequence is a polylinker and/or a sequence which enables integration of DNA sequences by recombination;
wherein the expression vector without the insertion sequence in total comprises at most 3000 bp; and
wherein the expression vector does not code for a regulator R₁ of P₁ nor for a regulator R₂ of P₂.

2. The expression vector according to Claim 1, **characterized in that** P₁ is a T7 promoter and/or P₂ is an a*ra* promoter.

3. The expression vector according to Claim 1 or 2, **characterized in that** it respectively codes for at least one terminator T₁ or T₂ in the corresponding reading direction of the promoters P₁ and P₂ respectively.

4. The expression vector according to Claim 3, **characterized in that** T₁ is a T7 terminator and/or T₂ is a terminator for the host RNA polymerase, or vice versa.

5. The expression vector according to Claim 3 or 4, **characterized in that** an additional gene lies between P₁ and its terminator T₁ in the reading direction of P₁ or that an additional gene lies between P₂ and its terminator T₂ in the reading direction of P₂.

6. An expression system containing an expression vector and a regulator vector, wherein the expression vector is defined as in one of Claims 1 to 5, and wherein the regulator vector codes for a regulator R₁ of P₁ and/or for a regulator R₂ of P₂.

7. The expression system according to Claim 6, **characterized in that** R₁ is *lacI* and/or R₂ is *araC*.

8. The expression system according to Claim 6 or 7, **characterized in that** the regulator vector additionally contains at least one gene for transfer RNAs of the host organism.

9. The expression system according to Claim 8, **characterized in that** the transfer RNA gene is selected from the group consisting of argU, argW, ileX, gluT, leuW, proL, metT, thrT, tyrU, thrU and argX of *E. coli,* which recognize the codons AGG, AGA, AUA, CUA, CCC, GGA and CGG.

10. The expression system according to Claim 9, **characterized in that** it contains the gene LysS for the T7 lysozyme.

11. A method for expression of DNA sequences with use of the expression vector according to one of Claims 1 to 5 or of the expression system according to one of Claims 6 to 10 comprising the steps
(i) Transfection or transformation of a suitable host organism with a regulator vector which codes for a regulator R₁ of P₁ and/or for a regulator R₂ of P₂;
(ii) Cloning of a DNA sequence or a DNA sequence mixture (library) into the expression vector between P₁ and P2;
(iii) Transfection or transformation of the host organism with the regulator vector obtained in (i) with the constructs obtained in step (ii); and
(iv) Induction of the expression of the proteins encoded by the DNA sequences by addition of an inducer I₁ for the regulator R₁ or an inducer I₂ for the regulator R₂.

12. A method for expression of DNA sequences with use of the expression vector according to one of Claims 1 to 5 or the expression system according to one of Claims 6 to 10 comprising the steps
a) Cloning of a DNA sequence or a DNA sequence mixture (library) into the expression vector between P₁ and P₂;
b) Transfection or transformation of a host organism, in which a regulator vector or parts thereof are integrated into the genome, which code for a regulator R₁ of P₁ and/or for a regulator R₂ of P₂, with the constructs obtained in step a); and
c) Induction of the expression of the proteins encoded by the DNA sequences by addition of an inducer I₁ for the regulator R₁ or an inducer I₂ for the regulator R₂.

13. The method according to one of Claims 11 or 12, **characterized in that** I₁ and I₂ are added to spatially separated part cultures of the host organisms with regulator vector and expression vector.

14. A method for screening DNA libraries with use of the expression vector according to one of Claims 1 to 5 or the expression system according to one of Claims 6 to 10 comprising the method according to one of Claims 11 to 13.

15. The method according to Claim 14, **characterized in that** the screening is effected with regard to a catalytic activity of the proteins expressed.

## Revendications

1. Vecteur d'expression comprenant deux promoteurs convergents P₁, P₂, inductibles indépendamment l'un de l'autre ;
dans lequel une séquence d'insertion est disposée entre P₁ et P₂, en aval de chacun d'eux, de telle sorte que l'expression d'une séquence d'ADN clonée dans la séquence d'insertion soit définie sous le contrôle de P₁ et de P₂ ;
la séquence d'insertion étant un lieur multisite et/ou une séquence qui permet une intégration de séquences d'ADN par recombinaison ;
le vecteur d'expression comprenant en tout, sans la séquence d'insertion, au plus 3000 pb ; et
le vecteur d'expression ne codant pas pour un régulateur R₁ de P₁ et ne codant pour un régulateur R₂ de P₂.

2. Vecteur d'expression selon la revendication 1, **caractérisé en ce que** P₁ est un promoteur T7 et/ou P₂ est un promoteur *Ara*.

3. Vecteur d'expression selon la revendication 1 ou 2, **caractérisé en ce qu'**il code pour au moins respectivement un terminateur T₁ et T₂, dans la direction de lecture correspondante des promoteurs respectivement P₁ et P₂.

4. Vecteur d'expression selon la revendication 3, **caractérisé en ce que** T₁ est un terminateur T7 et/ou T₂ est un terminateur pour l'ARN-polymérase hôte, ou inversement.

5. Vecteur d'expression selon la revendication 3 ou 4, **caractérisé en ce qu'**un gène supplémentaire se trouve entre P₁ et son terminateur T₁ dans la direction de lecture de P₁, ou qu'un gène supplémentaire se trouve entre P₂ et son terminateur T₂ dans la direction de lecture de P₂.

6. Système d'expression comprenant un vecteur d'expression et un vecteur régulateur, le vecteur d'expression étant défini comme dans l'une des revendications 1 à 5, et le vecteur régulateur codant pour un régulateur R₁ de P₁ et/ou pour un régulateur R₂ de P₂.

7. Système d'expression selon la revendication 6, **caractérisé en ce que** R₁ est *LacI* et/ou R₂ est *AraC.*

8. Système d'expression selon la revendication 6 ou 7, **caractérisé en ce que** le vecteur régulateur contient en outre au moins un gène pour l'ARN de transfert de l'organisme hôte.

9. Système d'expression selon la revendication 8, **caractérisé en ce que** le gène de l'ARN de transfert est choisi dans le groupe consistant en argU, argW, ileX, gluT, leuW, proL, metT, thrT, tyrU, thrU et ArgX de E. *coli,* qui reconnaissent les codons respectivement AGG, AGA, AUA, CUA, CCC, GGA et CGG.

10. Système d'expression selon la revendication 9, **caractérisé en ce qu'**il contient le gène LyS pour le lysozyme T7.

11. Procédé pour l'expression de séquences d'ADN par utilisation du vecteur d'expression selon l'une des revendications 1 à 5 ou du système d'expression selon l'une des revendications 6 à 10, comprenant les étapes
(i) transfection ou transformation d'un organisme hôte approprié avec un vecteur régulateur qui code pour un régulateur R₁ de P₁ et/ou pour un régulateur R₂ de P₂ ;
(ii) clonage d'une séquence d'ADN ou d'un mélange de séquences d'ADN (banque) dans le vecteur d'expression entre P₁ et P₂ ;
(iii) transfection ou transformation de l'organisme hôte obtenu en (i) avec un vecteur régulateur avec les constructions obtenues dans l'étape (ii) ; et
(iv) induction de l'expression des protéines codées par les séquences d'ADN par addition d'un inducteur I₁ pour le régulateur R₁ ou d'un inducteur I₂ pour le régulateur R₂.

12. Procédé pour l'expression de séquences d'ADN par utilisation du vecteur d'expression selon l'une des revendications 1 à 5 ou du système d'expression selon l'une des revendications 6 à 10, comprenant les étapes
a) clonage d'une séquence d'ADN ou d'un mélange de séquences d'ADN (banque) dans le vecteur d'expression entre P₁ et P₂ ;
b) transfection ou transformation d'un organisme hôte dans lequel un vecteur régulateur ou des parties de ce dernier sont intégrés dans le génome, qui codent pour un régulateur R₁ de P₁ et/ou pour un régulateur R₂ de P₂, avec les constructions obtenues dans l'étape a) ; et
c) induction de l'expression des protéines codées par les séquences d'ADN par addition d'un inducteur I₁ pour le régulateur R₁ ou d'un inducteur I₂ pour le régulateur R₂.

13. Procédé selon l'une des revendications 11 ou 12, **caractérisé en ce que** I₁ et I₂ sont ajoutés à des cultures partielles, spatialement séparées, des organismes hôtes obtenus, avec le vecteur régulateur et d'expression.

14. Procédé pour le criblage de banques d'ADN par utilisation du vecteur d'expression selon l'une des revendications 1 à 5 ou du système d'expression selon l'une des revendications 6 à 10, comprenant le procédé selon l'une des revendications 11 à 13.

15. Procédé selon la revendication 14, **caractérisé en ce que** le criblage est réalisé pour ce qui est d'une activité catalytique des protéines exprimées.
